(19) Europäisches Patentamt
European Patent Office
Office européen des brevets

(11) **EP 3 777 565 A1**

(12) **EUROPEAN PATENT APPLICATION**
published in accordance with Art. 153(4) EPC

(43) Date of publication:
**17.02.2021 Bulletin 2021/07**

(51) Int Cl.:
**A23L 29/262** (2016.01)  **A23L 2/42** (2006.01)
**A23L 2/62** (2006.01)  **A23L 29/00** (2016.01)
**A61K 47/36** (2006.01)  **A61K 47/38** (2006.01)

(21) Application number: **19781608.5**

(22) Date of filing: **29.03.2019**

(86) International application number:
**PCT/JP2019/013956**

(87) International publication number:
**WO 2019/194085 (10.10.2019 Gazette 2019/41)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **03.04.2018 JP 2018071778**

(71) Applicant: **Asahi Kasei Kabushiki Kaisha
Tokyo 1000006 (JP)**

(72) Inventors:
• **ASAI, Hirotaka
Tokyo 100-0006 (JP)**
• **YAMASAKI, Naoaki
Tokyo 100-0006 (JP)**
• **KAKIZAWA, Masayuki
Tokyo 100-0006 (JP)**
• **SAKAMOTO, Naoki
Tokyo 100-0006 (JP)**

(74) Representative: **Hoffmann Eitle
Patent- und Rechtsanwälte PartmbB
Arabellastraße 30
81925 München (DE)**

(54) **CELLULOSE POWDER**

(57)     The present invention provides a cellulose composite composed of a cellulose and a polysaccharide, wherein a Bragg spacing by small angle X-ray scattering (SAXS) of a sediment obtained by subjecting a 0.1 % by mass aqueous dispersion of the cellulose composite to a centrifugation treatment is 8.6 nm or more, and a method for producing the same.

*FIG. 1*

**Description**

TECHNICAL FIELD

The present invention relates to a cellulose composite composed of a cellulose and a polysaccharide, and a method for producing the same.

Priority is claimed on Japanese Patent Application No. 2018-071778, filed April 3, 2018, the content of which is incorporated herein by reference.

BACKGROUND ART

Conventionally, it is known that a cellulose composite of a cellulose and a polysaccharide forms a cellulose colloid in an aqueous medium and exhibits good suspension stability, and thus it is widely used in the fields of foods, pharmaceuticals, cosmetics, paints, ceramics, resins, catalysts, other industrial products and the like. The cellulose composite is used especially for the purpose of stabilizers such as a suspension stabilizer, an emulsion stabilizer, a thickening stabilizer or the like, a texture-imparting agent, a clouding agent, whiteness improvement, fluidity improvement, an abrasive, a dietary fiber, an oil and fat substitute or the like. For example, in the calcium-fortified milk, the cellulose composite is added for the purpose of improving the suspension stability of a water-insoluble component having a large specific gravity such as milk calcium and calcium carbonate. Further, in recent years, a large number of beverages and foods containing a large amount of water-insoluble components such as dietary fibers and proteins have been developed due to the increased interest in health and diet among the population. Particularly in beverages, when a large amount of these water-insoluble components are added, aggregation and sedimentation are likely to occur, so that a cellulose composite having high suspension-stabilizing performance has been desired.

Various studies have hitherto been made to improve the suspension stability of the cellulose composite. Patent Document 1 discloses a cellulose composite including a microcrystalline cellulose (MCC) and two kinds of carboxymethyl cellulose (CMC) having specific viscosities. It is described that the cellulose composite has a high storage elastic modulus and can stabilize cocoa particles in milk at a low additive amount (0.2% by mass). Further, Patent Document 2 discloses a dry powder of a cellulose composite having high gel strength, which is composed of MCC and CMC having two kinds of specific substitution degrees, and discloses that the cellulose composite exhibits high stabilizing performance with a small additive amount and is useful in various foods and drinks.

PRIOR ART LITERATURE

Patent Documents

   [Patent Document 1] WO 2013/022090
   [Patent Document 2] Japanese Unexamined Patent Application, First Publication No. 2015-502136

DISCLOSURE OF INVENTION

Problems to be Solved by the Invention

Conventional cellulose composite composed of a cellulose and a polysaccharide are dried, ground, and processed into powder form from the viewpoint of transportation and storage. However, it has been found that when the powdered cellulose composite is dispersed into solvents such as water, the composite structure is easily destroyed. For this reason, the conventional powdered cellulose composite did not function adequately as stabilizers, particularly in beverages containing a high concentration of insoluble components such as insoluble dietary fiber and proteins. In the beverages with a high concentration of insoluble components, homogenization at a long or high pressure is required, such as by a high-pressure homogenizer, because the shear energy in this homogenization process breaks the hydrogen bonds between the cellulose and the polysaccharide, and destroys the structure of the cellulose composite.

The cellulose composite described in Patent Document 1 is considered to have good suspending stability in a rich-tasting beverage with a high concentration of insoluble components, and it is described that the cellulose composite has excellent separation, agglomeration, and sedimentation in cocoa, coffee, and milk beverages containing black sesame at a concentration of 0.4 wt%. However, in this document, the cellulose composite was added to the beverage in a wet state without undergoing a drying process.

The dry powder of the cellulose composite described in Patent Document 2 can stabilize the calcium particles in the reduced milk at an additive amount of 0.3% by mass. However, the dry powder of the cellulose composite had a tendency to lower the gel strength of the aqueous dispersion in proportion to the shear energy applied when dispersed in water. For this reason, when added to foods and drinks that undergo shearing or homogenization in multiple stages in the manufacturing process, the stability of calcium particles will change due to the treatment of shearing and homogenizing, and it was difficult to determine the appropriate addition of the cellulose composite

The present invention is directed to providing a cellulose composite composed of a cellulose and a polysaccharide useful as a suspension stabilizer of insoluble components in liquid compositions such as beverages.

Means for Solving Problems

The present inventors have found that Bragg spacing of a sediment obtained by subjecting an aqueous dispersion of a composite composed of a cellulose and a polysaccharide to a centrifugation treatment is involved in the composite-formation strength of the cellulose and the polysaccharide. Furthermore, it was found that in a beverage to which the powder composition of the cellulose composite having a Bragg spacing of a specific value or more is added, regardless of the magnitude and amount of shear energy imparted upon the addition of said cellulose composite to the beverage, there is little change in the degree of separation, aggregation and sedimentation of beverage components, and thus the present invention has been completed. That is, the present invention is as follows.

[1] A cellulose composite comprising a cellulose and a polysaccharide, wherein
a Bragg spacing by small-angle X-ray scattering (SAXS) analysis of a sediment obtained by subjecting a 0.1% by mass aqueous dispersion of the cellulose composite to a centrifugation treatment is 8.6 nm or more.
[2] The cellulose composite according to [1], wherein a ratio ([G'2]/[G'1]) between

a storage elastic modulus (G'1) of an aqueous dispersion obtained by dispersing a mixture obtained by containing 1% by mass of the cellulose composite in ion-exchanged water using a high-shear homogenizer (manufactured by Nippon Seiki Co., Ltd., product name: "Excel Auto Homogenizer ED-7") at a rotation speed of 1,000 rpm for 5 minutes and
a storage elastic modulus (G'2) of an aqueous dispersion obtained by dispersing the mixture using the high-shear homogenizer at a rotation speed of 16,000 rpm for 5 minutes
is 0.5 or more.

[3] The cellulose composite according to [1] or [2], wherein the storage elastic modulus (G'2) of the aqueous dispersion obtained by dispersing a mixture obtained by containing 1% by mass of the cellulose composite in ion-exchanged water using a high-shear homogenizer (manufactured by Nippon Seiki Co., Ltd., product name: "Excel Auto Homogenizer ED-7") at a rotation speed of 16,000 rpm for 5 minutes is 0.3 Pa or more.
[4] The cellulose composite according to any one of [1] to [3], wherein

the cellulose composite has a water content of 20% by mass or less, and
the cellulose composite is in a powder form that passes through a sieve having an opening size of 1 mm.

[5] A method for producing a cellulose composite including a cellulose and a polysaccharide, comprising compositing a hydrolyzate of a cellulose having a Bragg spacing of 7.5 nm or more with a polysaccharide.
[6] A food and drink comprising the cellulose composite defined in any one of [1] to [4].
[7] A pharmaceutical product comprising the cellulose composite defined in any one of [1] to [4].
[8] An industrial product comprising the cellulose composite defined in any one of [1] to [4].

Effects of the Invention

The present invention provides a cellulose composite in which suspension stability does not easily change when blended in a liquid composition, regardless of the homogenization treatment time and shear stress applied. Therefore, it is possible to produce a food or drink product having both good suspension stability and smooth texture by adding the cellulose composite of the present invention to foods and beverages containing a high concentration of insoluble components such as cocoa particles, proteins and dietary fibers to homogenize with sufficient shear force.

BRIEF DESCRIPTION OF THE DRAWINGS

FIG. 1 shows the scattering intensity plots of the raw material pulps (LBKP pulp) after and before modifying treatment in Examples 1 and 3 (vertical axis: value $q^2I(q)$ obtained by multiplying scattering intensity after circular average by $q^2$, horizontal axis: logarithm of q).

FIG. 2 shows the scattering intensity plots of the sedimentation part of the dispersion liquid in Example 1 and Comparative Example 1 (vertical axis: value $q^2I(q)$ obtained by multiplying scattering intensity after circular average by $q^2$, horizontal axis: logarithm of q).

## BEST MODE FOR CARRYING OUT THE INVENTION

The present invention will be specifically described below.

The definition of the term "suspension-stable" in the present specification will be described. The term "suspension-stable" means that in a liquid composition containing an insoluble component in an aqueous medium, the insoluble component does not separate or settle and has a uniform appearance.

The cellulose composite according to the present invention is a cellulose composite composed of a cellulose and a polysaccharide, wherein a Bragg spacing by small-angle X-ray scattering (SAXS) analysis of a sediment obtained by subjecting a 0.1% by mass aqueous dispersion of the cellulose composite to a centrifugation treatment is 8.6 nm or more. In the present invention, the term "composite-formation of a cellulose and a polysaccharide" means that at least a part of the surface of the cellulose is coated with a polysaccharide by a chemical bond such as a hydrogen bond, and the term "cellulose composite" means a material in which at least a part of the surface of the cellulose is coated with a polysaccharide.

Cellulose obtained by hydrolyzing a cellulose raw material having a Bragg spacing by SAXS analysis of a specific value or more has a high accessibility of the hydroxyl groups in the cellulose molecule. Therefore, the cellulose obtained by hydrolyzing such a cellulose raw material forms many hydrogen bonds in the process of kneading and compositing with a polysaccharide, and a so-called high composite-formation strength can be achieved. As a result, the obtained cellulose composite exhibits high suspension stability performance, and the suspension stability performance does not deteriorate even when shearing is applied for a long time.

### <Cellulose>

The term "cellulose" in the present invention is a naturally-derived water-insoluble fibrous substance containing cellulose. Examples of the cellulose as a raw material of the cellulose composite according to the present invention include a cellulose obtained by subjecting a cellulose raw material such as wood pulp, non-wood pulp, straw, rice straw, cotton, cotton linter, hemp, ramie, bagasse, kenaf, beet, squirt, bacterial cellulose or the like to a hydrolysis treatment or the like. The cellulose raw material is preferably wood pulp or non-wood pulp, more preferably bleached wood pulp (BP), dissolving wood pulp (DP), cotton linter pulp, and most preferably wood bleached kraft pulp (BKP) and wood dissolved kraft pulp (DKP). These cellulose raw materials may be used alone or used as a mixture of two or more.

### <Bragg spacing of cellulose>

As the raw material of the cellulose composite according to the present invention, it is preferable to use a cellulose having a large Bragg spacing (d [nm]) in SAXS analysis. The Bragg spacing obtained by SAXS analysis represents the size of the cellulose microfibril gaps that constitute the cellulose. The larger the gaps of the cellulose microfibrils, the more sparse the structure of the cellulose and the higher the accessibility of the hydroxyl groups. Therefore, during the hydrolysis, it is easily attacked by an acid catalyst, and thus the degree of the polymerization is likely to decrease. Further, in the process of composite-formation with the polysaccharide by kneading, the hydrogen bonds are easily formed and composite-formation is promoted, which is preferable.

The Bragg spacing of cellulose can be measured by the following method. First, a mixture is prepared by adding ion-exchanged water to a cellulose sample having a dry weight of 30 g so that the total amount is 270 g. The mixture is defibrated (1,000 rpm×5 minutes) using a high-shear homogenizer (manufactured by Nippon Seiki Co., Ltd., product name "Excel Auto Homogenizer ED-7") to form a slurry. SAXS analysis is performed on the obtained slurry using a nanoscale X-ray structure evaluation apparatus (manufactured by Rigaku Corporation, product name "NANOPIX"). The analysis conditions are as follows. In addition, the sample should always be kept in a slurry state of being impregnated with water and should not be dried from the above operation to the end of the SAXS measurement. The solid content of the slurry is 10 to 50% by mass.

(SAXS analysis conditions)

X-ray wavelength λ: 0.154 nm
Measurement time: 900 seconds
Detector: Two-dimensional detector ("HyPix-6000")
Camera length: 1,307 mm

The one-dimensional scattering profile $I_{obs}$ (2θ) is obtained by circularly averaging the scattering pattern $I_{obs}$ (2θ, φ) measured by the two-dimensional detector under the above conditions as shown in Equation 1. In Equation 1, θ is the Bragg angle, P is the polarization factor, φ is the azimuth angle, and $I_{BG}$ is the detector background value.
[Mathematical formula 1]

$$I_{obs}(2\theta) = \frac{1}{2\pi P} \int_0^{2\pi} \frac{I_{obs}(2\theta, \phi) - I_{BG}}{\cos^3 2\theta} d\phi$$

1

Furthermore, the empty cell scattering correction is performed using Equation 2 for the one-dimensional profile obtained by circularly averaging. In Equation 2, I(2θ) is the scattering intensity after the empty cell scattering correction, $I_{obs}$ (2θ) is the uncorrected scattering intensity (the one-dimensional scattering profile obtained by Equation 1), and T is the X-ray transmittance of the sample. Further, the subscripts of "sample" and "empty" respectively indicate those obtained by the sample measurement and the empty cell measurement.
[Mathematical formula 2]

$$I(2\theta) = \frac{I_{obs,sample}(2\theta)}{T} - I_{obs,empty}(2\theta)$$

2

Next, using the absolute value q of the scattering vector represented by Equation 3, the data is plotted in such a manner that the vertical axis value is the value $q^2 I(q)$ obtained by multiplying the scattering intensity after circularly averaging by $q^2$ and the horizontal axis value is the logarithm of q, thereby determining the peak position $q_{max}$.
[Mathematical formula 3]

$$q = 4\pi \sin\theta / \lambda$$

3

Then, d [nm] calculated by the Bragg equation (Equation 4) using the $q_{max}$ is defined as the Bragg spacing.
[Mathematical formula 4]

$$d = \frac{\lambda}{2\sin\theta} = \frac{2\pi}{q_{max}}$$

4

When the Bragg spacing of the cellulose is 7.5 nm or more, the structure of the cellulose microfibrils is sufficiently sparse. The value of the Bragg spacing of the cellulose as a raw material of the cellulose composite according to the present invention is preferably 8.0 nm or more, more preferably 9.0 nm or more, and even more preferably 9.5 nm or more. The larger the Bragg spacing, the higher the accessibility of the hydroxyl groups of the cellulose molecule. Therefore, the upper limit is not particularly limited, but the preferable range is 25 nm or less in terms of the structure of the cellulose.

<Modification of cellulose>

Examples of the method for controlling the Bragg spacing of the cellulose include immersion in a solvent, blasting, beating and the like. These methods may be used alone or in combination of two or more. Further, these treatments may be carried out at any time before or after the hydrolysis of the cellulose for adjusting the average polymerization degree described later. By performing these treatments, hydrogen bonds inside and between the cellulose molecular chains are broken, and thus the accessibility to the hydroxyl groups is improved. Among the treatments, the solvent treatment of immersing in a solvent is preferable, and as the solvent, known solvents such as an alkaline aqueous solution, an ionic liquid, DMSO or the like can be used.

<Solvent treatment of cellulose>

By immersing in a solvent, impurities such as hemicellulose, lignin, resin and the like can be removed from the cellulose, and at the same time, hydrogen bonds between the cellulose molecular chains are destroyed. Therefore, the solvent treatment can further increase the Bragg spacing of the cellulose. Although the concentration of the slurry during the solvent treatment is not limited, by setting it to 1 to 20% by mass in terms of solid content, the entire cellulose sample can be uniformly treated. As the solvent used for the treatment, all known solvents for cellulose can be used. Among the solvents, ionic liquids, metal composite solutions, and alkaline aqueous solutions are preferable. In consideration of food safety, alkaline aqueous solutions are most preferable.

Here, the ionic liquid is defined as a substance having a melting point of 100°C or lower among the substances having an ionic bond. The type of the ionic liquid used for the solvent treatment is not limited and it may be used alone or in combination of two or more. Among the ionic liquids, imidazolium salts are preferable, and the treatment temperature is preferably 20 to 100°C, and the treatment time is preferably 1 to 60 minutes.

The metal composite solution is a solution containing a metal composite as a solute. The type of the metal composite used for the metal composite solution is not limited, and it may be used alone or in combination of two or more. As the metal composite solution used for the solvent treatment, $[Cu(NH_3)_4](OH)$ is preferable. At this time, the concentration ratio between Cu and $NH_3$ is preferably adjusted to $Cu/NH_3 = 1/6$ to $1/7$ [mol/mol]. The treatment temperature is preferably -20 to 100°C, and the treatment time is preferably about 1 to 60 minutes.

The type of the alkali used in the aqueous alkali solution is not limited, and it may be used alone or in combination of two or more. The alkali aqueous solution used for the solvent treatment is particularly preferably an alkali metal hydroxide (lithium hydroxide, sodium hydroxide, potassium hydroxide) aqueous solution, and most preferably a sodium hydroxide aqueous solution. It is preferable that the concentration of the sodium hydroxide be 1 to 11 % by mass, the treatment temperature be -10 to 80°C, and the treatment time be 1 to 300 minutes. By treating under these conditions, only the hydrogen bonds between the microfibrils are selectively broken, thereby making it possible to increase the Bragg spacing while maintaining the crystalline property of the cellulose in I-type.

It is considered that by performing any of the above-described treatments on the cellulose raw material, the solvent penetrates into the inside of the fibers of the cellulose raw material while maintaining the crystalline property of the cellulose, and thus the change in the cellulose structure occurs uniformly and the control of the composite-formation by kneading, which is a post-process, becomes easy.

<Average polymerization degree of cellulose>

The average polymerization degree of the cellulose as a raw material of the cellulose composite according to the present invention, is preferably 500 or less. Further, as the cellulose to be used, a crystalline cellulose is preferable. The average polymerization degree of the crystalline cellulose can be measured by a reduced specific viscosity method using a copper ethylenediamine solution. Specifically, the measurement is carried out in accordance with the procedure of the reduced specific viscosity method using a copper ethylenediamine solution defined in the crystalline cellulose confirmation test (3) of the "17th edition of the Japanese Pharmacopoeia", and if it exceeds the measurable range of the crystalline cellulose confirmation test (3), the measurement can be carried out in accordance with the procedure of the powdered cellulose confirmation test (3) of the same Japanese Pharmacopoeia. When the average polymerization degree is 500 or less, the crystalline cellulose is likely to be subjected to physical treatment such as stirring, pulverization and grinding in the composite-formation process with the polysaccharide, and thus the composite-formation is promoted, which is preferable. The average polymerization degree of the cellulose as a raw material is more preferably 400 or less, even more preferably 300 or less, still even more preferably 200 or less, and particularly preferably 150 or less. The lower the average polymerization degree, the easier the control of the composite-formation becomes. Therefore, the lower limit is not particularly limited, but a preferable range is 10 or more.

<Hydrolysis of cellulose>

Examples of the method for controlling the average polymerization degree of the cellulose include a hydrolysis treatment and the like. By the hydrolysis treatment, depolymerization of the amorphous cellulose inside the cellulose fiber progresses, and the average polymerization degree becomes small. At the same time, the hydrolysis treatment removes impurities such as hemicellulose, lignin or the like in addition to the above-mentioned amorphous cellulose, so that a crystalline cellulose with a high purity can be obtained. The method of hydrolysis is not particularly limited, and examples thereof include acid-catalyzed hydrolysis, alkali-catalyzed hydrolysis, hydrothermal decomposition and the like. These methods may be used alone or in combination of two or more. The hydrolysis method is preferably acid-catalyzed hydrolysis. The average polymerization degree can be easily controlled by adding an appropriate amount of an organic acid or an inorganic acid in a state where the cellulose is dispersed in an aqueous medium and heating the mixture while stirring. The reaction conditions such as temperature, pressure, time or the like at this time varies depending on the type of the cellulose, concentration of the cellulose, type of the acid and concentration of the acid, and can be appropriately adjusted so that the desired average polymerization degree is achieved. However, as the concentration of the acid catalyst becomes higher and the reaction temperature becomes higher, the hydrolysis of the acid catalyst such as hemicellulose and lignin is more likely to proceed, which causes coloring. Therefore, it is preferable that the concentration of the acid catalyst be low, and the reaction be performed at a low temperature.

Cellulose having a Bragg spacing of 7.5 nm or more has a large cellulose microfibril gap. Therefore, the accessibility of protons to the amorphous cellulose is high, and thereby it is possible to lower the acid concentration during the hydrolysis reaction and to lower the reaction temperature. Generally, an acid has a low swelling property on cellulose, and the hydrolysis reaction almost proceeds from the surface of cellulose. Therefore, in order to hydrolyze cellulose to LODP (level-off degree of polymerization) within a certain period of time, it is necessary to bring it into a high energy state under a high concentration of catalyst to sufficiently increase the reaction rate. However, since the cellulose having a large Bragg spacing and a sparse structure easily swells, protons penetrate into the inside of the microfibrils, and the hydrolysis proceeds rapidly. Therefore, the hydrolysis reaction proceeds sufficiently fast even under the conditions of low catalyst concentration and reaction energy. Therefore, when using a cellulose having a Bragg spacing of 7.5 nm or more, the hydrolysis conditions are preferably a hydrochloric acid concentration of 0.1 to 5.0% by mass and a reaction temperature of 70 to 170°C. The concentration of the hydrochloric acid is more preferably 1.5% by mass or less, and even more preferably 1.0% by mass or less. If the concentration of the hydrochloric acid is too low, the reaction time becomes long, and thus it is more preferably 0.3% by mass or more, and even more preferably 0.5% by mass or more.

<Crystalline property of cellulose>

The crystalline property of the cellulose as a raw material of the cellulose composite according to the present invention is preferably I-type crystal. Regarding the crystalline property, the fraction of I-type cellulose is calculated by an X-ray diffraction method (XRD) by the following procedure.

    1-1. Cellulose sample is prepared.
    1-2. Hydrochloric acid and water are added to the cellulose sample so that the final concentration is 2.5 N and the bath ratio is 10%, and the hydrolysis is performed at 105°C for 20 minutes while stirring at 100 rpm with a stirrer.
    1-3. The residue obtained is collected by suction filtration. At this time, the filtrate is washed and filtered with pure water until the pH of the filtrate becomes 4.0 to 7.0.
    1-4. The filtrate is dried in an oven at 105°C.
    1-5. The dried sample is crushed with a pestle and a mortar and the sample passed through a sieve having a mesh of 180 μm is used as a measurement sample (S1).
    1-6. Regarding S1, X-ray diffraction measurement is performed under the following conditions.

(XRD analysis conditions)

    Equipment (model): X-ray diffractometer (RINT-TTR)
    Measuring method: powder X-ray diffraction-measuring method
    X-ray wavelength: 0.15418nm (CU-K $\alpha$)
    Current: 50 kV
    Voltage: 200 mA
    Detector: Scintillation counter
    Divergence slit: 1 deg
    Scattering slit: 1 deg
    Light-receiving slit: 0.30 mm
    Scanning speed: 1.0 deg/min.
    Scan angle: 5 to 35°

After performing a smoothing process and background removal on the measurement results, the I-type crystallinity of cellulose (%) is defined as follows. If $I_{22.6}$ and $I_{18.5}$ are negative values after the smoothing process and background removal, they are defined as 0.

$$\text{I-type crystallinity of cellulose }(\%) = \{(I_{22.6} - I_{18.5})/ I_{22.6}\} \times 100$$

$I_{22.6}$ : Diffraction intensity of 002 plane (diffraction angle $2\theta = 22.6°$)
$I_{18.5}$ : Diffraction intensity of amorphous part (diffraction angle $2\theta = 18.5°$)

The smoothing process and background removal are performed under the following conditions.

(Smoothing process and background removal conditions)

Analysis software: Integrated powder X-ray analysis software PDXL
Smoothing method: Savitzky-Golay smoothing
Smoothing score: 11
Background removal method: Fitting method

When the I-type crystallinity of the cellulose is 40% or more, the hydrogen bonds are easily formed in the process of the composite-formation with the polysaccharide by kneading, and the composite-formation is promoted, which is preferable. The proportion of I-type crystallinity is preferably 70% or more, more preferably 85% or more. Even if the I-type crystallinity is very high, the accessibility to the hydroxyl groups of the cellulose of the polysaccharide is reduced, and thus it is difficult to proceed the composite-formation. Therefore, the I-type crystallinity is preferably 98% or less, more preferably 95% or less, and even more preferably 90% or less. The definition of cellulose I-type crystal is that a peak is recognized in the diffraction angle $2\theta$ of 22.1° to 23.1°.

<Compounding ratio of cellulose and polysaccharide>

The cellulose composite according to the present invention preferably contains 20 to 99% by mass of the cellulose and 1 to 80% by mass of the polysaccharide with respect to the total amount of the cellulose composite. By compositing the cellulose and the polysaccharide, the surface of the cellulose particles is coated with the polysaccharide by a chemical bond such as a hydrogen bond. The cellulose composite in which at least a part of the cellulose is coated with the polysaccharide can be dispersed in the aqueous dispersion, and the suspension stability is improved by forming a network structure. Compositing the cellulose and the polysaccharide with the above-described composition is preferable because the composite-formation effectively proceeds. The content of the cellulose with respect to the total amount of the cellulose composite is more preferably 60% by mass or more, even more preferably 70% by mass or more, and particularly preferably 75% by mass or more. If the ratio of the cellulose is too high, the dispersibility deteriorates. Therefore, the content of the cellulose with respect to the total amount of the cellulose composite is more preferably 95% by mass or less, and particularly preferably 90% by mass or less. The content of the polysaccharide with respect to the total amount of the cellulose composite is more preferably 5% by mass or more, and even more preferably 10% by mass or more. The content of the polysaccharide with respect to the total amount of the cellulose composite is more preferably 40% by mass or less, even more preferably 30% by mass or less, and particularly preferably 25% by mass or less.

<Polysaccharide>

The polysaccharide as a raw material of the cellulose composite of the present invention refers to a compound in which monosaccharides such as glucose, galactose, mannose, xylose, N-acetylglucosamine, gluconic acid, galacturonic acid and mannuronic acid or the like are connected via $\alpha$ or $\beta$ bond to constitute a main chain or a side chain. Examples of the naturally-derived polysaccharides include resin-derived polysaccharides such as almond gum, gum Arabic, arabinogalactan, elemi resin, gum karaya, gum ghatti, dammar resin, gum tragacanth, peach gum or the like; bean-derived polysaccharides such as linseed gum, cassia gum, locust bean gum, guar gum, an enzymatic decomposition product of guar gum, psyllium seed gum, Artemisia sphaerocephala seed gum, Sesbania seed gum, Tamarindus seed gum, tara gum, Triacanthos gum or the like; seaweed-derived polysaccharides such as alginic acid, carageenan, a Fukuronori extract, furcellaran or the like; polysaccharides derived from fruits, leaves and underground stems such as an aloe vera extract, an okra extract, a krantz aloe extract, Tororoaoi, pectin or the like; polysaccharides derived from microbial fermentation products such as Aeromonas gum, an Aureobasidium cultured solution, Azotobacter vinelandii gum, welan Gum, erwinia mitsuensis gum, Enterobacter simanus gum, Enterobacter gum, curdlan, xanthan gum, gellan gum, sklero gum, dextran, Bacillus natto gum, pullulan, Macrophomopsis gum, ramzan gum, levan or the like; cellulose-derived polysaccharides such as methylcellulose, ethylcellulose, hydroxyethylcellulose, hydroxypropylmethylcellulose, hydroxypropylcellulose, carboxymethylcellulose, carboxyethylcellulose or the like, and cellulose derivatives such as sodium salts, calcium salts thereof, and the like. Examples of other polysaccharides include yeast cell wall, chitin, chitosan, glucosamine, oligoglucosamine, heparin, chondroitin sulfate and the like. These polysaccharides may be used alone or in combination of two or more. Among these examples, as a raw material of the cellulose composite according to the present invention, a water-soluble polysaccharide is preferable, and further, anionic or neutral polysaccharides are preferable since they are easily formed into a composite with a crystalline cellulose. Since anionic polysaccharides are more easily formed into a composite, it is preferable that at least one anionic polysaccharide be blended in the cellulose composite according to the present invention.

<Anionic polysaccharide>

A polysaccharide which releases cations in water and serve as anions per se is called anionic polysaccharide. It is preferable to use an anionic polysaccharide as a raw material of the cellulose composite according to the present invention because the formation of the composite with cellulose is further promoted.

As the anionic polysaccharide, the following are preferable. Examples thereof include psyllium seed gum, karaya gum, carrageenan, agar, furcellaran, heparin, chondroitin sulfate, alginic acid, sodium alginate, calcium alginate, HM pectin, LM pectin, Azotobacter vinelandii gum, xanthan gum, gellan gum and a cellulose derivative such as carboxymethylcellulose sodium, carboxymethylcellulose calcium, sodium carboxyethylcellulose, calcium carboxyethylcellulose and the like. These anionic polysaccharides may be used alone or in combination of two or more. Among the examples, xanthan gum, gellan gum, carboxymethyl cellulose sodium, carboxymethyl cellulose calcium, carboxyethyl cellulose sodium, and carboxyethyl cellulose calcium are preferable since the composite-formation is promoted due to the structural similarity with the cellulose. Further, sodium carboxymethyl cellulose is particularly preferable, and it is even more particularly preferable if the molecular weight is 150,000 or less. The etherification degree of the carboxymethyl cellulose sodium is preferably 0.5-1.5, more preferably 0.86 to 1.5, and even more preferably 0.86 to 1.2. When the etherification degree is within this range, aggregation is unlikely to occur when added to a beverage.

<Cellulose composite>

The cellulose composite according to the present invention is a composite of a cellulose as a main component with a polysaccharide. As described above, the term "composite" means a form in which at least a part of the surface of the cellulose particles is coated with a polysaccharide by a chemical bond such as hydrogen bond. Therefore, the cellulose composite is not a state in which the cellulose powder and the polysaccharide are simply mixed, but a state in which the surface of the cellulose particles is covered with the polysaccharide. Therefore, when the cellulose composite is dispersed in an aqueous medium, the polysaccharide does not peel off from the surface of the cellulose particles, and forms a structure radially spreading from the surface, and becomes a colloid in water. This colloidal cellulose composite can form a high-order network structure by the interaction of electrostatic repulsion, steric repulsion, van der Waals force, and the like.

<Bragg spacing of cellulose composite>

The cellulose composite according to the present invention has a characteristic that Bragg spacing is large. The Bragg spacing of the cellulose composite is a Bragg spacing of the cellulose particles that form a core of the composite, and it can be measured by the following method.

2-1. First, the cellulose composite is weighed (for example, so that the total amount is 300 g), and mixed with ion-exchanged water so that the content of the cellulose composite is 0.1% by mass, and the obtained mixture is stirred using the Excel Auto Homogenizer at 15,000 rpm for 5 minutes.
2-2. The obtained aqueous dispersion is dispensed (for example, 30 mL per bottle) into a stoppered test tube for a centrifuge (manufactured by KUBOTA Corporation, product name "Compact high-speed cooling centrifuge 6930", rotor: RA-400, 8×50 mL), and centrifuged at 8,000 G (G represents gravitational acceleration) for 10 minutes with the device.
2-3. After centrifugation, the supernatant is removed by decanting, then 25 mL of ion-exchanged water is added to the sedimentation part, the mixture is stirred with a vortex mixer until the sedimentation is resolved, and centrifugation is performed again at 8,000 G for 10 minutes.

2-4. The operation 2-3 is repeated.

2-5. The above procedures 2-1 to 2-4 is repeated to obtain 0.5 g of a sediment. Regarding the sediment, the Bragg spacing of the cellulose composite can be measured by SAXS analysis using a nanoscale X-ray structure evaluation device (manufactured by Rigaku Corporation, product name "NANOPIX") in the same manner as the measurement of the Bragg spacing of cellulose.

The sample should always be kept in a slurry state of being impregnated with water from the operations 2-1 to 2-5 to the end of SAXS measurement, and should not dried. The solid content of the slurry is 10 to 50% by mass.

By the above operations 2-1 to 2-4, cellulose particles contained in the aqueous dispersion of the cellulose composite which have an appropriate size for the SAXS analysis can be recovered. The Bragg spacing obtained by the SAXS analysis 1-5 is a size of the gap of the cellulose microfibrils forming the coarse particles, and this structure is maintained even in the crystalline cellulose particles as the core of the cellulose composite. That is, since the accessibility of hydroxyl groups is high in the cellulose having a large value of Bragg spacing, a large number of water molecules are present in the microfibril gaps and mediate a large number of hydrogen bonds between the crystalline cellulose and the water-soluble polysaccharide. In other words, the larger the Bragg spacing of the coarse particles, the more strongly are the cellulose and water-soluble polysaccharide formed into composite. Therefore, even when a high shear force is applied, the hydrogen bond between the crystalline cellulose and the water-soluble polysaccharide is not broken, and the functions of the cellulose composite such as suspension-stabilizing ability or the like are not easily impaired.

When the Bragg spacing of the cellulose composite is 8.6 nm or more, the cellulose particles and the polysaccharide can be sufficiently strongly formed into composite. The Bragg spacing of the cellulose composite according to the present invention is preferably 9.0 nm or more. Among them, it is preferably 9.5 nm or more, more preferably 10.0 nm or more, even more preferably 10.5 nm or more, particularly preferably 11.0 nm or more, more particularly preferably 11.5 nm or more, even more particularly preferably 12.0 nm, and most preferably 12.5 nm or more. Although the upper limit of the Bragg spacing of the cellulose composite is not particularly limited, considering the structure of the cellulose itself, the Bragg spacing of the cellulose composite is preferably 50 nm or less. The Bragg spacing of the cellulose composite tends to be larger than that of the cellulose used as a raw material. This is because the molecular weight of the cellulose decreases in the hydrolysis and kneading processes, and water molecules easily enter the microfibril gaps.

<Composite-formation strength of cellulose composite *Ratio of storage elastic modulus>

The cellulose composite according to the present invention is strongly formed into a composite. The composite-formation degree can be considered as the ratio of the hydrogen bond between the cellulose and the polysaccharide. As the composite-formation progresses, the ratio of the hydrogen bond between the cellulose and the polysaccharide in the cellulose composite increases, and the effect of the present invention improves.

The composite-formation strength of the cellulose composite is defined as follows. The composite-formation strength of the cellulose composite is a ratio ([G'2]/[G'1]) of a storage elastic modulus (G'2) when dispersing the cellulose composite contained in ion-exchanged water in an amount of 1% by mass at a high speed (processing condition: rotation speed of 16,000 rpm×5 minutes) using a high-shear homogenizer (manufactured by Nippon Seiki Co., Ltd., product name: "Excel Auto Homogenizer ED-7") to a storage elastic modulus (G'1) when dispersing at a low speed (processing condition: rotation speed of 1,000 rpm×5 minutes). The storage elastic modulus (G'1) and the storage elastic modulus (G'2) are measured as follows.

3-1. The cellulose composite is dispersed in pure water at a low speed (processing condition: rotation speed of 1,000 rpm×5 minutes) using a high-shear homogenizer (manufactured by Nippon Seiki Co., Ltd., product name "Excel Auto Homogenizer ED-7") to prepare a 1.0% by mass pure water dispersion. The obtained aqueous dispersion is allowed to stand in a water bath at 25°C for 24 hours.

3-2. The strain dependence of the stress of the water dispersion is measured using a viscoelasticity-measuring device (manufactured by TA Instrument, ARES-G2 type, geometry: Double Wall Couette type). 20 g of the aqueous dispersion is charged at a rate of 20 g/min or less using a dropper so as not to destroy the fine structure, and allowed to stand for 5 minutes, and then measurement is started in the Dynamic Strain mode (temperature: constantly 25.0°C, Angular velocity: 20 rad/sec, strain: sweeping in the range of 1 →794%). In the present invention, as the storage elastic modulus (G'1), the value of 20% strain on the strain-stress curve obtained by the above measurement is used.

3 -3. The cellulose composite was dispersed in pure water at a high speed (processing condition: rotation speed 16,000 rpm×5 minutes) using a high-shear homogenizer (Nippon Seiki Co., Ltd., product name "Excel Auto Homogenizer ED-7") to prepare a 1.0% by mass pure water dispersion. The obtained aqueous dispersion is allowed to stand at room temperature for 24 hours.

3-4. The strain dependence of the stress of the water dispersion is measured using a viscoelasticity-measuring device (manufactured by TA Instrument, ARES-G2 type, geometry: Double Wall Couette type). 20 g of the aqueous dispersion is charged at a rate of 20 g/min or less using a dropper so as not to destroy the fine structure, and allowed to stand for 5 minutes, and then measurement is started in the Dynamic Strain mode (temperature: constantly 25.0°C, Angular velocity: 20 rad/sec, strain: sweeping in the range of 1→794%). In the present invention, as the storage elastic modulus (G'2), the value of 20% strain on the strain-stress curve obtained by the above measurement is used.

The value of [G'2]/[G'1] of the cellulose composite according to the present invention is preferably 0.5 or more. When the value of [G'2]/[G'1] is 0.5 or more, it can be said that the composite-formation strength of the cellulose composite is sufficiently high. The value of [G'2]/[G'1] of the cellulose composite according to the present invention is more preferably 0.7 or more, even more preferably 0.85 or more, still even more preferably 0.90 or more, and particularly preferably 0.95 or more. Since the stability during the manufacturing process of food and drink does not deteriorate by the large value of [G'2]/[G'1], the upper limit is not particularly set. However, if it is too large, the dispersibility tends to deteriorate, and for this reason, in order to disperse the cellulose composite in a liquid composition and exert the suspension stability performance, a larger shear energy is required. Therefore, the value of [G'2]/[G'1] of the cellulose composite according to the present invention is preferably 10 or less, more preferably 5 or less, even more preferably 2 or less, still even more preferably 1.5 or less, and particularly preferably 1.2 or less.

<Storage elastic modulus of cellulose composite>

The cellulose composite of the present invention preferably has a storage elastic modulus (G'2) of 0.3 Pa or more when dispersing 1% by mass of the cellulose composite in ion-exchanged water at a high speed (processing conditions: rotation speed 16,000 rpm x 5 minutes) with a high-shear homogenizer (Nippon Seiki Co., Ltd. product name "Excel Auto Homogenizer ED-7").

The value of G'2 of the cellulose composite in the present invention is preferably 0.3 Pa or more. If the value of G'2 is 0.3 Pa or more, it is considered that the cellulose and the polysaccharide form a good composite, and when dispersed in a solvent, due to the interaction between the cellulose composites, a rigid gel can be formed to exhibit sufficient suspension stability. This means that the larger this value, the greater the number of the cellulose composites, and the aqueous dispersion becomes a stiffer gel because the cellulose composites form more interactions with each other.

The storage elastic modulus of the cellulose composite is preferably 0.45 Pa or more, more preferably 1.0 Pa or more, even more preferably 1.5 Pa or more, and most preferably 3.0 Pa or more. The upper limit is not particularly set, and the larger this value, the smaller the ratio of the cellulose composite added to the beverage.

<Crystalline property of cellulose contained in cellulose composite>

The crystalline property of the cellulose contained in the cellulose composite according to the present invention is preferably I-type crystal. Regarding the crystalline property, the fraction of I-type cellulose is calculated by an X-ray diffraction method (XRD) by the following procedure.

4-1. Cellulose composite sample is prepared.
4-2. Hydrochloric acid and water are added to the cellulose composite sample so that the final concentration is 2.5 N and the bath ratio is 10%, and the hydrolysis is performed at 105°C for 20 minutes while stirring at 100 rpm with a stirrer.
4-3. The residue obtained is collected by suction filtration. At this time, the filtrate is washed and filtered with pure water until the pH of the filtrate becomes 4.0 to 7.0.
4-4. The filtrate is dried in an oven at 105°C to adjust the water content to 2 to 10%. 4-5. The dried sample is crushed with a pestle and a mortar and the sample passed through a sieve having a mesh of 180 $\mu$m is used as a measurement sample (S1).
4-6. Regarding S1, X-ray diffraction measurement is performed under the following conditions.

(XRD analysis conditions)

Equipment (model): X-ray diffractometer (RTNT-TTR)
Measuring method: powder X-ray diffraction-measuring method
X-ray wavelength: 0.15418 nm (CU-K $\alpha$)

Current: 50kV
Voltage: 200mA
Detector: Scintillation counter
Divergence slit: 1 deg
Scattering slit: 1 deg
Light-receiving slit: 0.30 mm
Scanning speed: 1.0 deg/min.
Scan angle: 5 to 35°

After performing a smoothing process and background removal on the measurement results, the I-type crystallinity of cellulose (%) is defined as follows. If $I_{22.6}$ and $I_{18.5}$ are negative values after the smoothing process and background removal, they are defined as 0.

$$\text{I-type crystallinity of cellulose (\%)} = \{(I_{22.6} - I_{18.5})/I_{22.6}\} \times 100$$

$I_{22.6}$ : Diffraction intensity of 002 plane (diffraction angle $2\theta = 22.6°$)
$I_{18.5}$ : Diffraction intensity of amorphous part (diffraction angle $2\theta = 18.5°$)

The smoothing process and background removal are performed under the following conditions.

(Smoothing process and background removal conditions)

Analysis software: Integrated powder X-ray analysis software PDXL
Smoothing method: Savitzky-Golay smoothing
Smoothing score: 11
Background removal method: Fitting method

When the I-type crystallinity of the cellulose is 40% or more, the composite-formation is promoted in the process of composite-formation with the polysaccharide by kneading, and thus the amount of the uncomposited polysaccharide, so-called free polysaccharide is reduced, which is preferable. The proportion of the I-type crystallinity is preferably 70% or more, more preferably 85% or more. When the I-type crystallinity is too high, the composite-formation does not proceed sufficiently in the kneading process, and the amount of the free polysaccharide increases. Therefore, the I-type crystallinity is preferably 98% or less, more preferably 95% or less, and even more preferably 90% or less. The definition of cellulose I-type crystal is that a peak is recognized in the diffraction angle $2\theta$ of 22.1° to 23.1°.

<Composite-formation of cellulose and polysaccharide>

The cellulose composite having a specific Bragg spacing according to the present invention is obtained by using a cellulose having a specific value or more of Bragg spacing as a raw material and applying a mechanical shear force to the cellulose and the polysaccharide in the kneading process to make the crystalline cellulose finer, while forming a composite with the polysaccharide on the cellulose surface. At the time of kneading, a water-soluble gum, a hydrophilic substance, and other additives may be added in addition to the polysaccharide.
To apply a mechanical shear force, a kneading method using a kneading machine or the like can be used. As the kneading machine, a kneader, an extruder, a planetary mixer, a liquor machine or the like can be used, and they may be a continuous type or a batch type. These models can be used alone, or in combination of two or more. These models may be appropriately selected depending on the viscosity requirement in various applications.
The temperature during the kneading may be arbitrary, and if heat is generated due to the composite-formation reaction or friction during kneading, the kneading may be performed while removing the heat. In the production of the cellulose composite according to the present invention, the kneading temperature is preferably low. This is because the lower the kneading temperature is, the more the deterioration of the polysaccharide is suppressed, and as a result, the storage modulus (G') of the resulting cellulose composite becomes higher. The kneading temperature when kneading the cellulose and the polysaccharide is preferably 80°C or lower, more preferably 70°C or lower, even more preferably 60°C or lower, still even more preferably 50°C or lower, particularly preferably 30°C or lower, and most preferably 20°C or lower. In order to maintain the above kneading temperature under a high energy, it is also possible to use jacket cooling, gradual heating such as heat radiation.

The solid content at the time of kneading the cellulose and the polysaccharide is preferably 35% by mass or more. By kneading the kneading object having a high viscosity in a semi-solid state, the kneading energy is easily transferred to the kneading object without the kneading object becoming loose, and the composite-formation is promoted, which is preferable. The solid content at the time of kneading is more preferably 40% by mass or more, even more preferably 50% by mass or more, and still even more preferably 55% by mass or more. Although the upper limit of the solid content at the time of kneading is not particularly limited, considering that a sufficient kneading effect and a uniform kneading state can be obtained without causing the kneading object to have a dry state with a small amount of water, the practical range is preferably 90% by mass or less, more preferably 70% by mass or less, even more preferably 60% by mass or less. Further, in order to adjust the solid content to the above range, for the timing of water addition, a necessary amount may be added before the kneading process, during the kneading process, or at both timings.

<Drying Method of Cellulose Composite>

In obtaining the powder of the cellulose composite according to the present invention, when drying the kneaded product obtained from the above-mentioned kneading process, known drying methods such as tray drying, spray drying, belt drying, fluidized bed drying, freeze drying, microwave drying or the like may be used. When the kneaded product is subjected to the drying process, it is preferable to maintain the solid concentration in the kneading process without adding water to the kneaded product. The water content of the dried cellulose composite is preferably 1 to 20% by mass. By setting the water content to 20% by mass or less, it is possible to prevent spoilage occurring when stored at room temperature, and problems such as stickiness and spoilage, or problems of cost in conveyance and transportation are less likely to occur. The water content of the dried cellulose composite is more preferably 10% by mass or less, even more preferably 6% by mass or less. Further, when the water content is 1% by mass or more, deterioration of dispersibility due to excessive drying can be suppressed. The water content is more preferably 1.5% by mass or more.

When the cellulose composite is distributed on the market, since the powder-form is easier to handle, it is preferable to pulverize the cellulose composite obtained by drying to form a powder-form. However, when the spray drying is used as the drying method, since the drying and powdering can be performed at the same time, pulverization is not necessary. When pulverizing the dried cellulose composite, known methods such as a cutter mill, a hammer mill, a pin mill, a jet mill or the like can be used. The pulverization degree is preferably such that the pulverized product can pass through a sieve having an opening size of 1 mm, and more preferably pass through a sieve having an opening size of 425 $\mu$m and have an average particle diameter (weight-average particle diameter) of 10 to 250 $\mu$m. The powdery dried cellulose composite having such a size is easily dispersed when stirred in water, and therefore, a stable colloidal dispersion with smooth texture in which cellulose is uniformly dispersed can be formed. In particular, in the neutral state, the cellulose does not cause aggregation or separation and forms a stable colloidal dispersion, and therefore, the cellulose composite has an excellent function as a stabilizer or the like.

<Intended use>

The cellulose composite according to the present invention can be used in various types of foods, industrial products and pharmaceuticals. Examples of the foods and drinks capable of blending with the cellulose composite according to the present invention include various beverages such as tasty beverages such as coffee, tea, powdered green tea, cocoa, sweet red bean soup, juice or the like, milk beverages such as raw milk, processed milk, lactic acid bacteria beverages, soybean milk or the like, nutrition-enriched beverages such as calcium-enriched beverages or the like, dietary fiber-containing beverages or the like; ice confectioneries such as ice cream, ice milk, soft cream, milk shake, sherbet or the like; dairy products such as butter, cheese, yogurt, coffee whitener, whipping cream, custard cream, puddings or the like; processed oils and fats such as mayonnaise, margarine, spread, shortening or the like; liquid foods such as various soups, stews or the like; liquid seasonings such as sauces, dipping sauces, dressings or the like; various spice paste represented by mustard paste; fillings represented by jam and flour paste; gel paste foods such as bean paste and jelly; solid foods such as cereal foods such as bread, noodle, pasta, pizza, premix or the like; Japanese and western confectioneries such as candy, cookie, biscuit, pancake, chocolate, rice cake or the like; fish paste products represented by fish sausage, Hanppen Japanese fish sausage or the like; livestock products represented by ham, sausage, hamburger steak or the like; prepared foods such as cream croquette, filling for Chinese food, gratin, dumpling or the like; food delicacies such as salted fish guts, pickles with lees or the like; pet foods; fluid foods for tube feeding; and the like.

In these uses, the cellulose composite of the present invention serves as a calorie-reducing base, such as a suspension stabilizer, an emulsification stabilizer, a thickening stabilizer, a foam stabilizer, a clouding agent, a texture-imparting agent, a fluidity improver, a shape-retaining agent, a syneresis inhibitor, a dough modifier, a powder base, a dietary fiber base and oil and fat alternative. Furthermore, the effects of the present invention can be exhibited even if the form of the foods or drinks containing the cellulose composite according to the present invention and the processing method for preparation at the time of use are different. That is, the cellulose composite according to the present invention has an excellent suspension-stabilizing effect even when blended in a retort food, a powdered food, a frozen food, a food for a microwave oven, and the like. Particularly, the cellulose composite according to the present invention is different from the conventional cellulose-based materials in that it functions even in a heating environment and a high-concentration environment. In particular, by blending the cellulose composite according to the present invention, the suspension stability of the insoluble component is remarkably improved, and the problem of a grainy feeling can be overcome by its smooth feeling on the tongue and body. Therefore, the cellulose composite can be used in a wide variety of foods other than those described above.

<Method for adding cellulose composite>

A food product using the cellulose composite of the present invention may be prepared by adding a main raw material, if necessary, by blending a flavor, a pH moderator, a thickening stabilizer, salts, saccharides, fats and oil, proteins, an emulsifier, an acidulant and a dye, and applying an operation such as mixing, kneading, stirring, emulsifying, heating or the like by use of the same apparatus as generally used in producing foods. Specifically, as a method for adding the cellulose composite of the present invention to a food and drink, the following method is mentioned. For example, the cellulose composite of the present invention can be added by dispersing it in aqueous media such as water simultaneously with a main raw-material or other components such as a coloring agent, a spice, an acidulant, a thickener or the like. Furthermore, when the dry powder of the cellulose composite is dispersed in an aqueous medium, it is preferable that the cellulose composite be once dispersed in water and then added to a desired food form. This is because the suspension stability of the cellulose composite is improved. When the cellulose composite is dry powder, the cellulose composite can be dispersed in water by a method of using a kneading machine including various types of dispersion machines, emulsifiers and grinders usually used in production processes of foods. Specific examples of the kneading machine that can be used include various types of mixers such as a propeller stirrer, a high-speed mixer, a homo mixer and a cutter; mills such as a ball mill, a colloidal mill, a beads mill and a grinder (Raikai mixer); dispersers/emulsifiers represented by high-pressure homogenizer such as high-pressure homogenizer and a nanomizer; and kneading machines represented by e.g., a planetary mixer, kneader, extruder and turbulizer. The kneading machines may be used alone or in combination of two types or more. Furthermore, dispersion can be easily made if kneading is performed while increasing the temperature.

<Additive amount of cellulose composite to food and drink>

The additive amount of cellulose composite to a food and drink is not particularly limited. For example, the additive amount of cellulose composite to beverages such as coffee, cocoa and milk is preferably 0.01 mass% or more. If the additive amount of cellulose composite is 0.01 mass% or more, dispersion and suspension stability of the insoluble components in the food and drink increases, and excellent emulsion stability and a syneresis prevention effect can be obtained. The additive amount of cellulose composite to the food and drink is more preferably 0.05 mass% or more and further preferably 0.1 mass% or more. If the additive amount of cellulose composite is 5 mass% or less, aggregation and separation of the cellulose composite do not occur. Also, in view of ease to take as a beverage (feeling in the throat, grainy feeling on the tongue), the additive amount is preferably 5 mass% or less.
In the foods and beverages containing a high concentration of insoluble components, it is necessary to homogenize them with a sufficient shear force in order to obtain a smooth texture and enhance the digestibility of the insoluble components. However, in the conventional cellulose composite, when the shear energy becomes large, the suspension-stabilizing ability decreases, and therefore, when blending and homogenizing it with a high shear force, it becomes a food or drink with a low suspension stability. On the other hand, the suspension stability of the cellulose composite according to the present invention is less likely to be affected by the shear energy, and therefore, the suspension stability is unlikely to change even if the time of the homogenization process and shear stress are changed to adjust the texture and digestibility. Therefore, when the cellulose composite according to the present invention is blended in a food or drink as a suspension stabilizer, it is possible to adjust the digestibility and texture of the insoluble components by adjusting the shear force in the homogenization process of dispersing the cellulose composite, and as a result, the manufacturing process of the foods or beverages can be simplified. In addition, even when homogenizing with a high shear force to smooth the texture, it is not necessary to change the additive amount of the crystalline cellulose to obtain the required suspension stability, and the texture can be adjusted without causing taste change.

<Applications other than food and drink>

The cellulose composite of the present invention is significantly improved in the strength of cellulose composite, and particularly, in a composition of an aqueous suspension state containing a water-insoluble component, a stable dispersion state can be maintained without causing aggregation, separation, syneresis and sedimentation. For this reason, it can be used for various purposes other than foods and drinks. For example, other than foods and drinks, the cellulose composite of the present invention can be blended with medicinal products such as a syrup agent, a liquid agent and an ointment; cosmetics such as a lotion, an emulsion and a cleaner; cleaners for food and industrial use, raw materials for cleaners and treatment agents for food and industrial use, raw materials for detergents for household use (clothes, a kitchen, a house, tableware, etc.), paints, pigments, ceramics, water based latex, agents for emulsification (polymerization), agents for agriculture, agents for fiber processing (refinement agent, dyeing assistant, softener, water repellent), soil-release finishing agents, concrete admixtures, printing inks, lubrication oils, antistatic agents, antifog additives, lubricants, dispersants, deinking agents, etc.

[EXAMPLES]

The present invention will be explained by way of the examples below. However, these examples do not limit the scope of the present invention.

<Bragg spacing of cellulose>

In the following Examples and the like, the Bragg spacing of cellulose was measured as follows.

First, ion-exchanged water was added to a cellulose sample having a dry weight of 30 g so that the total amount was 270 g. The resulting mixture was defibrated (1,000 rpm×5 minutes) with a high-shear homogenizer (manufactured by Nippon Seiki Co., Ltd., product name: "Excel Auto Homogenizer ED-7") to form a slurry. SAXS analysis was performed on the obtained slurry using a nanoscale X-ray structure evaluation apparatus (manufactured by Rigaku Corporation, product name: "NANOPIX") under the following conditions. The sample was kept in a water-impregnated slurry state from the mixing of the dry cellulose sample with ion-exchanged water until the end of SAXS measurement, so as not to be dried. The solid content of the slurry was set to about 10 to 50% by mass.

(SAXS analysis conditions)

    X-ray wavelength $\lambda$: 0.154 nm
    Measurement time: 900 seconds
    Detector: Two-dimensional detector ("HyPix-6000")
    Camera length: 1,307 mm

The one-dimensional scattering profile $I_{obs}$ (2$\theta$) was obtained by circularly averaging the scattering pattern $I_{obs}$ (2$\theta$, $\varphi$) measured by the two-dimensional detector under the above conditions as shown in Equation 1. In Equation 1, $\theta$ is the Bragg angle, P is the polarization factor, $\varphi$ is the azimuth angle, and $I_{BG}$ is the detector background value.

[Mathematical formula 5]

$$I_{obs}(2\theta) = \frac{1}{2\pi P}\int_0^{2\pi} \frac{I_{obs}(2\theta,\phi) - I_{BG}}{\cos^3 2\theta}d\phi \qquad\qquad 1$$

Furthermore, an empty cell scattering correction was performed using Equation 2 for the circularly averaged one-dimensional profile. In Equation 2, I(2$\theta$) is the scattering intensity after the empty cell scattering correction, $I_{obs}$ (2$\theta$) is the uncorrected scattering intensity (the one-dimensional scattering profile obtained by Equation 1), and T is the X-ray transmittance of the sample. Further, the subscripts of "sample" and "empty" indicate those obtained by the sample measurement and the empty cell measurement, respectively.

[Mathematical formula 6]

$$I(2\theta) = \frac{I_{obs,sample}(2\theta)}{T} - I_{obs,empty}(2\theta) \qquad\qquad 2$$

Next, using the absolute value q of the scattering vector represented by Equation 3, the data was plotted in such a manner that the vertical axis value is the value $q^2I(q)$ obtained by multiplying the scattering intensity after circularly averaging by $q^2$ and the horizontal axis value is the logarithm of q, thereby determining the peak position $q_{max}$.
[Mathematical formula 7]

$$q = 4\pi \sin \theta / \lambda$$

$$3$$

Then, d [nm] calculated by the Bragg equation (Equation 4) using the $q_{max}$ was defined as the Bragg spacing.
[Mathematical formula 8]

$$d = \frac{\lambda}{2 \sin \theta} = \frac{2\pi}{q_{max}}$$

$$4$$

<Crystalline property of cellulose>

In the following examples and the like, the crystalline property of cellulose was measured as follows.
First, 26 g of a cellulose sample was measured, and hydrochloric acid and water were added thereto so that the final concentration was 2.5 N and the bath ratio was 10%. The resulting mixture was heated to 105°C using an oil bath while stirring with a stirrer, and after reaching 105°C, hydrolysis was performed for 20 minutes. The obtained slurry was filtered and washed with pure water until the pH became 4.0 or higher. Further, the obtained hydrolysis residue was dried in an oven at 105°C until the water content became 2 to 10%. The dried sample was crushed using a pestle and a mortar, and the sample that passed through a sieve with a mesh of 180 $\mu$m was used as a sample for measurement, followed by subjecting the sample for measurement to XRD analysis under the following conditions.

(XRD analysis conditions)

Equipment (model): X-ray diffractometer (RINT-TTR)
Measuring method: powder X-ray diffraction measuring method
X-ray wavelength: 0.15418 nm (CU-K $\alpha$)
Current: 50 kV
Voltage: 200 mA
Detector: Scintillation counter
Divergence slit: 1 deg
Scattering slit: 1 deg
Light receiving slit: 0.30 mm
Scanning speed: 1.0 deg/min.
Scan angle: 5 to 35°

After performing a smoothing process and background removal on the measurement results, the I-type crystallinity of cellulose (%) was calculated as follows.

$$\text{I-type crystallinity of cellulose (\%)} = \{(I_{22.6} - I_{18.5}) / I_{22.6}\} \times 100$$

$I_{22.6}$ : Diffraction intensity of 002 plane (diffraction angle $2\theta$ = 22.6°)
$I_{18.5}$ : Diffraction intensity of amorphous part (diffraction angle $2\theta$ = 18.5°)

(Smoothing process and background removal conditions)

Analysis software: Integrated powder X-ray analysis software PDXL
Smoothing method: Savitzky-Golay smoothing
Smoothing score: 11
Background removal method: Fitting method

<Bragg spacing of cellulose composite>

In the following Examples and the like, the Bragg spacing of the cellulose composite was measured as follows.

First, ion-exchanged water and the cellulose composite were weighed so that the content of the cellulose composite was 0.1% by mass and the total amount was 300 g, followed by mixing. The resulting mixture was stirred at 15,000 rpm for 5 minutes using the above-described Excel Auto Homogenizer. The obtained water dispersion was dispensed into stoppered test tubes for a centrifuge (KUBOTA Corporation, product name "Compact high-speed cooling centrifuge 6930", rotor: RA-400, 8×50 mL) in an amount of 30 mL per tube, followed by centrifuging at 8,000 G with this device for 10 minutes. After the centrifugation, the supernatant was removed by decanting, 25 mL of ion-exchanged water was added to the sedimentation part and stirred with a vortex mixer until the sedimentation was resolved, followed by centrifuging again at 8,000 G for 10 minutes to wash the sediment. This washing operation was repeated twice. The above operation was repeated, and the obtained sediment was subjected to SAXS analysis using the nanoscale X-ray structure evaluation device (manufactured by Rigaku Corporation, product name "NANOPIX") under the same conditions as described in the section <Bragg spacing of cellulose> to calculate the Bragg spacing (d [nm]). The sample was kept in a slurry state of being constantly impregnated with water from the mixing of ion-exchanged water and the cellulose composite until the end of the SAXS measurement, so as not to be dried. The solid content of the slurry was 10 to 50% by mass.

<Crystalline Property of Cellulose Composite>

In the following examples and the like, the crystalline property of the cellulose were measured as follows.

First, 26 g of a cellulose sample was measured, and hydrochloric acid and water were added thereto so that the final concentration was 2.5 N and the bath ratio was 10%. The resulting mixture was heated to 105°C using an oil bath while stirring with a stirrer, and after reaching 105°C, hydrolysis was performed for 20 minutes. The obtained slurry was filtered and washed with pure water until the pH became 4.0 or higher. Further, the obtained hydrolysis residue was dried in an oven at 105°C until the water content became 2 to 10%. The dried sample was crushed using a pestle and a mortar and the sample that passed through a sieve with a mesh of 180 $\mu$m was used as a sample for measurement, followed by subjecting the sample for measurement to XRD analysis under the following conditions.

<Composite-formation strength of cellulose composite>

The composite-formation strength of the cellulose composite is represented by the ratio ([G'2]/[G'1]) of the storage elastic modulus (G'2) to the storage elastic modulus (G'1). In the following examples and the like, the storage elastic modulus (G'1) and the storage elastic modulus (G'2) were measured as follows.

First, the cellulose composite was dispersed in pure water (processing condition: rotation speed of 1,000 rpm×5 minutes) using a high-shear homogenizer (manufactured by Nippon Seiki Co., Ltd., product name "Excel Auto Homogenizer ED-7") to prepare a 1.0% by mass aqueous dispersion 1. The obtained aqueous dispersion 1 was allowed to stand at room temperature for 24 hours. The strain dependence of the stress of the water dispersion 1 was measured using a viscoelasticity-measuring device (manufactured by TA Instrument, ARES-G2 type, geometry: Double Wall Couette type). 20 g of the aqueous dispersion was charged at a rate of 20 g/min or less using a dropper so as not to destroy the fine structure, and allowed to stand for 5 minutes, and then measurement was started in the Dynamic Strain mode (temperature: constantly 25.0°C, Angular velocity: 20 rad/sec, strain: sweeping in the range of 1→794%). As the storage elastic modulus (G'1), the value of 20% strain on the strain-stress curve obtained by the above measurement was used.

Separately, the cellulose composite was dispersed in pure water (processing condition: rotation speed of 16,000 rpm×5 minutes) using a high-shear homogenizer (manufactured by Nippon Seiki Co., Ltd., product name "Excel Auto Homogenizer ED-7") to prepare a 1.0% by mass aqueous dispersion 2. The obtained aqueous dispersion 2 was allowed to stand at room temperature for 24 hours. The strain dependence of the stress of the water dispersion 2 was measured using a viscoelasticity-measuring device (manufactured by TA Instrument, ARES-G2 type, geometry: Double Wall Couette type). 20 g of the aqueous dispersion was charged at a rate of 20 g/min or less using a dropper so as not to destroy the fine structure, and allowed to stand for 5 minutes, and then measurement was started in the Dynamic Strain mode (temperature: constantly 25.0°C, Angular velocity: 20 rad/sec, strain: sweeping in the range of 1→794%). As the storage elastic modulus (G'2), the value of 20% strain on the strain-stress curve obtained by the above measurement was used.

[Example 1]

A commercially available bleached hardwood kraft pulp (LBKP) was shredded, and then added to a 7 mass% NaOHaq whose temperature was adjusted to 4°C so that the solid concentration was 9 mass%, followed by stirring for 1 hour. Then, the resulting mixture was filtered and washed with water until the filtrate showed neutrality to obtain purified pulp (modified cellulose).

The purified pulp thus prepared was hydrolyzed in hydrochloric acid having a final concentration of 0.65% by mass at 121 °C for 60 minutes, and the resulting hydrolyzate was washed with water and filtered to prepare a wet cake-shaped cellulose (MCC) having a solid content of 43% by mass. Next, the MCC and the CMC-Na (substitution degree of 0.93) were charged into a biaxial kneader (manufactured by DSM Xplore, product name "Compounder 15") so that MCC:CMC-Na = 85:15, and the solid concentration was 42 mass% (adjusted with ion-exchanged water), followed by wet-kneading at a kneading temperature of 30°C, 50 rpm for 10 minutes. The kneaded product thus prepared was dried in an oven at 100°C for 30 minutes, then pulverized by a centrifugal pulverizer (manufactured by Retsch, product name "ZM200") with a screen having an opening size of 0.75 mm, and then further sieved using a sieve having an opening size of 0.18 mm to obtain a powder that passed through the sieve as cellulose composite powder composition A.

The LBKP used as a raw material had a Bragg spacing of 7.2 nm, the modified cellulose had a Bragg spacing of 9.8 nm, and the I-type crystallinity was 88%. In addition, the water content of the resulting cellulose composite powder composition A was 5.8% by mass, the Bragg spacing of the sedimentation part of the aqueous dispersion was 12.9 nm, the storage elastic modulus (G'1) was 2.90, the storage elastic rate (G'2) was 3.41, and therefore [G'2]/[G'1]=1.18, and the I-type crystallinity was 89%. The results are shown in Table 1.

[Example 2]

A commercially available bleached hardwood kraft pulp (LBKP) was shredded, and then added to a 6 mass% NaOHaq whose temperature was adjusted to 4°C so that the solid concentration was 9 mass%, followed by stirring for 1 hour. Then, the resulting mixture was filtered and washed with water until the filtrate showed neutrality to obtain purified pulp (modified cellulose).

The purified pulp thus prepared was hydrolyzed in hydrochloric acid having a final concentration of 0.65% by mass at 121 °C for 60 minutes, and the resulting hydrolyzate was washed with water and filtered to prepare a wet cake-shaped cellulose (MCC) having a solid content of 43% by mass. Next, the MCC and the CMC-Na (substitution degree of 0.93) were charged into a biaxial kneader (manufactured by DSM Xplore, product name "Compounder 15") so that MCC:CMC-Na = 85:15, and the solid concentration was 42 mass% (adjusted with ion-exchanged water), followed by wet-kneading at a kneading temperature of 70°C, 50 rpm for 10 minutes. The kneaded product thus prepared was dried in an oven at 100°C for 30 minutes, then pulverized by a centrifugal pulverizer (manufactured by Retsch, product name "ZM200") with a screen having an opening size of 0.75 mm, and then further sieved using a sieve having an opening size of 0.18 mm to obtain a powder that passed through the sieve as cellulose composite powder composition B.

The LBKP used as a raw material had a Bragg spacing of 7.2 nm, the modified cellulose had a Bragg spacing of 9.7 nm, and the I-type crystallinity was 91%. In addition, the water content of the resulting cellulose composite powder composition B was 9.1% by mass, the Bragg spacing of the sedimentation part of the aqueous dispersion was 12.7 nm, the storage elastic modulus (G'1) was 1.52, the storage elastic rate (G'2) was 1.69, and therefore [G'2]/[G'1]=1.11, and the I-type crystallinity was 91%. The results are shown in Table 1.

[Example 3]

A commercially available bleached hardwood kraft pulp (LBKP) was shredded, and then added to a 9 mass% NaOHaq whose temperature was adjusted to 30°C so that the solid concentration was 9 mass%, followed by stirring for 1 hour. Then, the resulting mixture was filtered and washed with water until the filtrate showed neutrality to obtain purified pulp (modified cellulose).

The purified pulp thus prepared was hydrolyzed in hydrochloric acid having a final concentration of 0.65% by mass at 121 °C for 60 minutes, and the resulting hydrolyzate was washed with water and filtered to prepare a wet cake-shaped cellulose (MCC) having a solid content of 44% by mass. Next, the MCC and the CMC-Na (substitution degree of 0.93) were charged into a biaxial kneader (manufactured by DSM Xplore, product name "Compounder 15") so that MCC:CMC-Na = 85:15, and the solid concentration was 42 mass% (adjusted with ion-exchanged water), followed by wet-kneading at a kneading temperature of 70°C, 50 rpm for 10 minutes. The kneaded product thus prepared was dried in an oven at 100°C for 30 minutes, then pulverized by a centrifugal pulverizer (manufactured by Retsch, product name "ZM200") with a screen having an opening size of 0.75 mm, and then further sieved using a sieve having an opening size of 0.18 mm to obtain a powder that passed through the sieve as cellulose composite powder composition C.

The LBKP used as a raw material had a Bragg spacing of 7.2 nm, the modified cellulose had a Bragg spacing of 9.5 nm, and the I-type crystallinity was 93%. In addition, the water content of the resulting cellulose composite powder composition C was 7.1% by mass, the Bragg spacing of the sedimentation part of the aqueous dispersion was 12.3 nm, the storage elastic modulus (G'1) was 1.40, the storage elastic rate (G'2) was 1.35, and therefore [G'2]/[G'1]=0.96, and the I-type crystallinity was 92%. The results are shown in Table 1.

[Example 4]

A commercially available bleached hardwood kraft pulp (LBKP) was shredded, and then added to a 5 mass% NaOHaq whose temperature was adjusted to 4°C so that the solid concentration was 9 mass%, followed by stirring for 1 hour. Then, the resulting mixture was filtered and washed with water until the filtrate showed neutrality to obtain purified pulp (modified cellulose).

The purified pulp thus prepared was hydrolyzed in hydrochloric acid having a final concentration of 0.65% by mass at 121 °C for 60 minutes, and the resulting hydrolyzate was washed with water and filtered to prepare a wet cake-shaped cellulose (MCC) having a solid content of 43% by mass. Next, the MCC and the CMC-Na (substitution degree of 0.93) were charged into a biaxial kneader (manufactured by DSM Xplore, product name "Compounder 15") so that MCC:CMC-Na = 85:15, and the solid concentration was 42 mass% (adjusted with ion-exchanged water), followed by wet-kneading at a kneading temperature of 30°C, 50 rpm for 10 minutes. The kneaded product thus prepared was dried in an oven at 100°C for 30 minutes, then pulverized by a centrifugal pulverizer (manufactured by Retsch, product name "ZM200") with a screen having an opening size of 0.75 mm, and then further sieved using a sieve having an opening size of 0.18 mm to obtain a powder that passed through the sieve as cellulose composite powder composition D.

The LBKP used as a raw material had a Bragg spacing of 7.2 nm, the modified cellulose had a Bragg spacing of 8.9 nm, and the I-type crystallinity was 95%. In addition, the water content of the resulting cellulose composite powder composition D was 10.8% by mass, the Bragg spacing of the sedimentation part of the aqueous dispersion was 11.2 nm, the storage elastic modulus (G'1) was 0.99, the storage elastic rate (G'2) was 0.65, and therefore [G'2]/[G'1]=0.66, and the I-type crystallinity was 95%. The results are shown in Table 1.

[Example 5]

A commercially available bleached hardwood kraft pulp (LBKP) was shredded, and then added to a 4 mass% NaOHaq whose temperature was adjusted to 4°C so that the solid concentration was 9 mass%, followed by stirring for 1 hour. Then, the resulting mixture was filtered and washed with water until the filtrate showed neutrality to obtain purified pulp (modified cellulose).

The purified pulp thus prepared was hydrolyzed in hydrochloric acid having a final concentration of 0.65% by mass at 121°C for 60 minutes, and the resulting hydrolyzate was washed with water and filtered to prepare a wet cake-shaped cellulose (MCC) having a solid content of 42% by mass. Next, the MCC and the CMC-Na (substitution degree of 0.93) were charged into a biaxial kneader (manufactured by DSM Xplore, product name "Compounder 15") so that MCC:CMC-Na = 85:15, and the solid concentration was 42 mass% (adjusted with ion-exchanged water), followed by wet-kneading at a kneading temperature of 30°C, 50 rpm for 10 minutes. The kneaded product thus prepared was dried in an oven at 100°C for 30 minutes, then pulverized by a centrifugal pulverizer (manufactured by Retsch, product name "ZM200") with a screen having an opening size of 0.75 mm, and then further sieved using a sieve having an opening size of 0.18 mm to obtain a powder that passed through the sieve as cellulose composite powder composition E.

The LBKP used as a raw material had a Bragg spacing of 7.2 nm, the modified cellulose had a Bragg spacing of 8.0 nm, and the I-type crystallinity was 97%. In addition, the water content of the resulting cellulose composite powder composition E was 6.2% by mass, the Bragg spacing of the sedimentation part of the aqueous dispersion was 9.8 nm, the storage elastic modulus (G'1) was 0.77, the storage elastic rate (G'2) was 0.48, and therefore [G'2]/[G'1]=0.62, and the I-type crystallinity was 97%. The results are shown in Table 1.

[Example 6]

A commercially available bleached hardwood kraft pulp (LBKP) was shredded, and then added to $H_2O$ so that the solid concentration was 9 mass%, followed by heating and stirring at 150°C in a pressure vessel for 1 hour. Then, the resulting mixture was filtered to obtain purified pulp (modified cellulose).

The purified pulp thus prepared was hydrolyzed in hydrochloric acid having a final concentration of 0.65% by mass at 121°C for 60 minutes, and the resulting hydrolyzate was washed with water and filtered to prepare a wet cake-shaped cellulose (MCC) having a solid content of 42% by mass. Next, the MCC and the CMC-Na (substitution degree of 0.93) were charged into a biaxial kneader (manufactured by DSM Xplore, product name "Compounder 15") so that MCC:CMC-Na = 85:15, and the solid concentration was 42 mass% (adjusted with ion-exchanged water), followed by wet-kneading at a kneading temperature of 30°C, 50 rpm for 10 minutes. The kneaded product thus prepared was dried in an oven at 100°C for 30 minutes, then pulverized by a centrifugal pulverizer (manufactured by Retsch, product name "ZM200") with a screen having an opening size of 0.75 mm, and then further sieved using a sieve having an opening size of 0.18 mm to obtain a powder that passed through the sieve as cellulose composite powder composition F.

The LBKP used as a raw material had a Bragg spacing of 7.2 nm, the modified cellulose had a Bragg spacing of 7.6 nm, and the I-type crystallinity was 98%. In addition, the water content of the resulting cellulose composite powder composition F was 4.9% by mass, the Bragg spacing of the sedimentation part of the aqueous dispersion was 8.8 nm, the storage elastic modulus (G'1) was 0.62, the storage elastic rate (G'2) was 0.31, and therefore [G'2]/[G'1]=0.50, and the I-type crystallinity was 97%. The results are shown in Table 1.

[Example 7]

A commercially available hardwood dissolved sulfide pulp (LDSP) was shredded, and then added to a 7 mass% NaOHaq whose temperature was adjusted to 4°C so that the solid concentration was 9 mass%, followed by stirring for 1 hour. Then, the resulting mixture was filtered and washed with water until the filtrate showed neutrality to obtain purified pulp (modified cellulose).

The purified pulp thus prepared was hydrolyzed in hydrochloric acid having a final concentration of 0.65% by mass at 121°C for 60 minutes, and the resulting hydrolyzate was washed with water and filtered to prepare a wet cake-shaped cellulose (MCC) having a solid content of 42% by mass. Next, the MCC and the CMC-Na (substitution degree of 0.93) were charged into a biaxial kneader (manufactured by DSM Xplore, product name "Compounder 15") so that MCC:CMC-Na = 85:15, and the solid concentration was 42 mass% (adjusted with ion-exchanged water), followed by wet-kneading at a kneading temperature of 30°C, 50 rpm for 10 minutes. The kneaded product thus prepared was dried in an oven at 100°C for 30 minutes, then pulverized by a centrifugal pulverizer (manufactured by Retsch, product name "ZM200") with a screen having an opening size of 0.75 mm, and then further sieved using a sieve having an opening size of 0.18 mm to obtain a powder that passed through the sieve as cellulose composite powder composition G.

The LDSP used as a raw material had a Bragg spacing of 7.2 nm, the modified cellulose had a Bragg spacing of 9.7 nm, and the I-type crystallinity was 90%. In addition, the water content of the resulting cellulose composite powder composition G was 7.5% by mass, the Bragg spacing of the sedimentation part of the aqueous dispersion was 12.7 nm, the storage elastic modulus (G'1) was 3.12, the storage elastic rate (G'2) was 3.40, and therefore [G'2]/[G'1]=1.09, and the I-type crystallinity was 89%. The results are shown in Table 1.

[Example 8]

A commercially available bleached hardwood kraft pulp (LBKP) was shredded, and then added to 1-ethyl-3-methylimidazolium diethyl phosphate (manufactured by Nippon Emulsifier Co., Ltd.) whose temperature was adjusted to 80°C so that the solid concentration was 9% by mass, followed by stirring for 10 minutes. Thereafter, centrifugation was performed at 8,000 G for 5 minutes using a centrifuge (manufactured by KUBOTA Corporation, product name "Compact High Speed Cooling Centrifuge 6930"), followed by removing the supernatant by decanting. An excess amount of ethanol was added to the obtained sediment, the resulting mixture was centrifuged again and the supernatant was removed to replace the solvent. This replacement operation was performed twice more, namely, a total of 3 times, and then the pulp washing operation was performed twice more by the same procedure using ion-exchanged water at 30°C to obtain purified pulp (modified cellulose).

The purified pulp thus prepared was hydrolyzed in hydrochloric acid having a final concentration of 0.65% by mass at 121°C for 60 minutes, and the resulting hydrolyzate was washed with water and filtered to prepare a wet cake-shaped cellulose (MCC) having a solid content of 43% by mass. Next, the MCC and the CMC-Na (substitution degree of 0.93) were charged into a biaxial kneader (manufactured by DSM Xplore, product name "Compounder 15") so that MCC:CMC-Na = 85:15, and the solid concentration was 42 mass% (adjusted with ion-exchanged water), followed by wet-kneading at a kneading temperature of 30°C, 50 rpm for 10 minutes. The kneaded product thus prepared was dried in an oven at 100°C for 30 minutes, then pulverized by a centrifugal pulverizer (manufactured by Retsch, product name "ZM200") with a screen having an opening size of 0.75 mm, and then further sieved using a sieve having an opening size of 0.18 mm to obtain a powder that passed through the sieve as cellulose composite powder composition H.

The LBKP used as a raw material had a Bragg spacing of 7.2 nm, the modified cellulose had a Bragg spacing of 9.7 nm, and the 1-type crystallinity was 86%. In addition, the water content of the resulting cellulose composite powder composition H was 7.2% by mass, the Bragg spacing of the sedimentation part of the aqueous dispersion was 12.4 nm, the storage elastic modulus (G'1) was 2.49, the storage elastic rate (G'2) was 2.58, and therefore [G'2]/[G'1]=1.04, and the I-type crystallinity was 85%. The results are shown in Table 1.

[Comparative Example 1]

A commercially available bleached hardwood kraft pulp (LBKP) was shredded, and then hydrolyzed in hydrochloric acid having a final concentration of 0.65% by mass at 121°C for 60 minutes, and the resulting hydrolyzate was washed with water and filtered to prepare a wet cake-shaped cellulose (MCC) having a solid content of 45% by mass. Next, the MCC and the CMC-Na (substitution degree of 0.93) were charged into a biaxial kneader (manufactured by DSM Xplore, product name "Compounder 15") so that MCC:CMC-Na = 85:15, and the solid concentration was 42 mass% (adjusted with ion-exchanged water), followed by wet-kneading at a kneading temperature of 30°C, 50 rpm for 10 minutes. The kneaded product thus prepared was dried in an oven at 100°C for 30 minutes, then pulverized by a centrifugal pulverizer (manufactured by Retsch, product name "ZM200") with a screen having an opening size of 0.75 mm, and then further sieved using a sieve having an opening size of 0.18 mm to obtain a powder that passed through the sieve as cellulose composite powder composition I.

The LBKP used as a raw material had a Bragg spacing of 7.2 nm, and the I-type crystallinity was 99%. In addition, the water content of the resulting cellulose composite powder composition I was 6.5% by mass, the Bragg spacing of the sedimentation part of the aqueous dispersion was 8.3 nm, the storage elastic modulus (G'1) was 0.31, the storage elastic rate (G'2) was 0.14, and therefore [G'2]/[G'1]=0.45, and the 1-type crystallinity was 99%. The results are shown in Table 2.

[Comparative Example 2]

A commercially available hardwood dissolved sulfide pulp (LDSP) was hydrolyzed in hydrochloric acid having a final concentration of 0.65% by mass at 121°C for 60 minutes, and the resulting hydrolyzate was washed with water and filtered to prepare a wet cake-shaped cellulose (MCC) having a solid content of 49% by mass. Next, the MCC and the CMC-Na (substitution degree of 0.93) were charged into a biaxial kneader (manufactured by DSM Xplore, product name "Compounder 15") so that MCC:CMC-Na = 85:15, and the solid concentration was 46 mass% (adjusted with ion-exchanged water), followed by wet-kneading at a kneading temperature of 30°C, 50 rpm for 10 minutes. The kneaded product thus prepared was dried in an oven at 100°C for 30 minutes, then pulverized by a centrifugal pulverizer (manufactured by Retsch, product name "ZM200") with a screen having an opening size of 0.75 mm, and then further sieved using a sieve having an opening size of 0.18 mm to obtain a powder that passed through the sieve as cellulose composite powder composition J.

The LDSP used as a raw material had a Bragg spacing of 7.2 nm, and the I-type crystallinity was 99%. In addition, the water content of the resulting cellulose composite powder composition J was 5.2% by mass, the Bragg spacing of the sedimentation part of the aqueous dispersion was 8.5 nm, the storage elastic modulus (G'1) was 1.24, the storage elastic rate (G'2) was 0.58, and therefore [G'2]/[G'1]=0.47, and the I-type crystallinity was 99%. The results are shown in Table 2.

[Comparative Example 3]

A commercially available hardwood dissolved sulfide pulp (LDSP) was hydrolyzed in hydrochloric acid having a final concentration of 0.65% by mass at 121°C for 60 minutes, and the resulting hydrolyzate was washed with water and filtered to prepare a wet cake-shaped cellulose (MCC) having a solid content of 49% by mass. Next, the MCC and the CMC-Na (substitution degree of 0.93) were charged into a biaxial kneader (manufactured by DSM Xplore, product name "Compounder 15") so that MCC:CMC-Na = 85:15, and the solid concentration was 50 mass% (adjusted with ion-exchanged water), followed by wet-kneading at a kneading temperature of 30°C, 50 rpm for 30 minutes. The kneaded product thus prepared was dried in an oven at 100°C for 30 minutes, then pulverized by a centrifugal pulverizer (manufactured by Retsch, product name "ZM200") with a screen having an opening size of 0.75 mm, and then further sieved using a sieve having an opening size of 0.18 mm to obtain a powder that passed through the sieve as cellulose composite powder composition K.

The LDSP used as a raw material had a Bragg spacing of 7.2 nm, and the I-type crystallinity was 99%. In addition, the water content of the resulting cellulose composite powder composition K was 4.5% by mass, the Bragg spacing of the sedimentation part of the aqueous dispersion was 8.4 nm, the storage elastic modulus (G'1) was 2.66, the storage elastic rate (G'2) was 1.31, and therefore [G'2]/[G'1]=0.49, and the I-type crystallinity was 99%. The results are shown in Table 2.

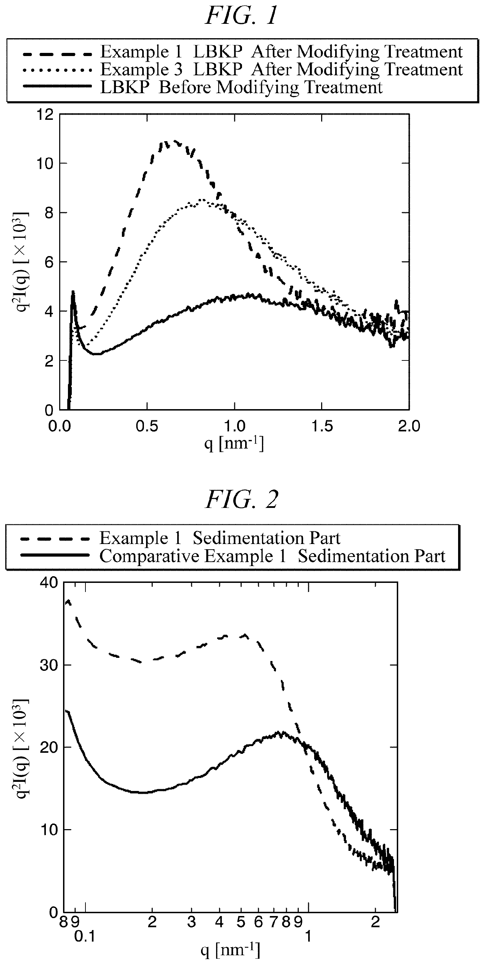

FIG. 1 shows the scattering intensity plots of the raw material pulps (LBKP pulp) before and after the modifying treatment in Examples 1 and 3. Further, FIG. 2 shows the scattering intensity plots of the sedimentation part of the dispersion liquid in Example 1 and Comparative Example 1. In each case, the vertical axis represents the value $q^2I(q)$ obtained by multiplying the scattering intensity after circular average by $q^2$, and the horizontal axis represents the logarithm of q.

[Table 1]

| | | Ex. 1 | Ex. 2 | Ex. 3 | Ex. 4 | Ex. 5 | Ex. 6 | Ex. 7 | Ex. 8 |
|---|---|---|---|---|---|---|---|---|---|
| Prescription | Cellulose raw material | LBKP | LBKP | LBKP | LBKP | LBKP | LBKP | LDSP | LBKP |
| | d value [nm] of cellulose | 7.2 | 7.2 | 7.2 | 7.2 | 7.2 | 7.2 | 7.2 | 7.2 |
| | Modification treatment | 7%, 4°C, NaOHaq | 6%, 4°C, NaOHaq | 9%, 30°C, NaOHaq | 5%, 4°C, NaOHaq | 4%, 4°C, NaOHaq | 150°C, $H_2O$ | 7%, 4°C, NaOHaq | Imidazolium, 80°C |
| | d value [nm] of modified cellulose | 9.8 | 9.7 | 9.5 | 8.9 | 8.0 | 7.6 | 9.7 | 9.7 |
| | I-type crystallinity after modification [%] | 88 | 91 | 93 | 95 | 97 | 98 | 90 | 86 |
| | CMC-Na | 15 | 15 | 15 | 15 | 15 | 15 | 15 | 15 |
| Physical properties of composite | Water content [%] | 5.8 | 9.1 | 7.1 | 10.8 | 6.2 | 4.9 | 7.5 | 7.2 |
| | d value of sedimentation part of dispersion | 12.9 | 12.7 | 12.3 | 11.2 | 9.8 | 8.8 | 12.7 | 12.4 |
| | G'1 (1,000 rpm, after 24h) [Pa] | 2.90 | 1.52 | 1.4 | 0.99 | 0.77 | 0.62 | 3.12 | 2.49 |
| | G'2 (16,000 rpm, after 24h) [Pa] | 3.41 | 1.69 | 1.35 | 0.65 | 0.48 | 0.31 | 3.40 | 2.58 |
| | G'2/G'1 | 1.18 | 1.11 | 0.96 | 0.66 | 0.62 | 0.5 | 1.09 | 1.04 |
| | I-type crystallinity [%] | 89 | 91 | 92 | 95 | 97 | 97 | 89 | 85 |

[Table 2]

| | | Comp.Ex. 1 | Comp.Ex. 2 | Comp.Ex. 3 |
|---|---|---|---|---|
| Prescription | Cellulose raw material | LBKP | LDSP | LDSP |
| | d value [nm] of cellulose | 7.2 | 7.2 | 7.2 |
| | I-type crystallinity after modification [%] | 99 | 99 | 99 |
| | Modification treatment | - | - | - |
| | d value [nm] of modified cellulose | - | - | - |
| | Cellulose | 85 | 85 | 85 |
| | CMC-Na | 15 | 15 | 15 |
| Physical properties of composite | Water content [%] | 6.5 | 5.2 | 4.5 |
| | d value of sedimentation part of dispersion | 8.3 | 8.5 | 8.4 |
| | G'1 (1,000 rpm, after 24h) [Pa] | 0.31 | 1.24 | 2.66 |
| | G'2 (16,000 rpm, after 24h) [Pa] | 0.14 | 0.58 | 1.31 |
| | G'2/G'1 | 0.45 | 0.47 | 0.49 |
| | I-type crystallinity [%] | 99 | 99 | 99 |

<Carrot juice containing high concentration of insoluble dietary fiber>

Using the cellulose composite powder compositions A to K obtained in the above Examples and Comparative Examples, two kinds of carrot juice having different textures were prepared by the following operations and evaluated.

First, 15 g of sugar and an appropriate amount of the cellulose composite powder composition were added to 300 g of grated carrots and 300 g of apples, and ion-exchanged water was further added so as to adjust the total amount to 1,000 g. After stirring the resulting mixture with a spatula, using a piston type homogenizer (manufactured by APV, product name: "Manton Gaulin Homogenizer"), homogenization treatment was performed at 10 MPa to obtain a carrot juice (rough), and performed at 40 MPa 10 times to obtain a carrot juice (smooth). These were filled in a glass heat-resistant bottle having a volume of 200 mL, heat-sterilized (121°C, 30 minutes), and cooled with tap water for 1 hour. The cooled bottle was gently shaken up and down 10 times and then stored in an atmosphere of 5°C. for 1 month.

The suspension stability of each carrot juice after storage was visually observed to evaluate the separation state and the sedimentation state. Also, the carrot juice after storage was drunk and the texture was sensory evaluated. The evaluation method was performed based on the following criteria for the separation state, sedimentation state, the number of re-dispersion and the texture. The evaluation results are shown in Tables 3 to 6.

(Separation state)

The ratio of the light-colored upper layer of the beverage in the heat-resistant bottle to the entire beverage was measured.

◎ (excellent): No separation (no light-colored layer was observed)
○ (good): Light-colored layer was less than 10% of the whole beverage
△ (acceptable): Light-colored layer was less than 30% of the whole beverage
✕ (unacceptable): Light-colored layer was 30% or more of the whole beverage

(Sedimentation state)

The beverage in the heat-resistant bottle was slowly turned upside down and evaluated based on the amount of deposited substance on the bottom surface.

◎ (excellent): no deposits on the bottom
○ (good): deposits were thinly attached on a part of the bottom surface
△ (acceptable): deposits were thinly attached on the entire bottom surface
✕ (unacceptable): deposits were thickly attached on the entire bottom surface

(Number of re-distribution)

The beverage in the heat-resistant bottle was slowly rotated, and the number of times until the deposits on the bottom surface were completely eliminated was used to evaluate.

(Texture)

Smooth: the texture was smooth on the palate
Rough: the texture was rough on the palate

<Red bean milk drink>

Using the cellulose composite powder compositions A to K obtained in the above Examples and Comparative Examples, two kinds of red bean milk beverages having different textures were prepared by the following operations and evaluated.

First, 100 g of sugar-free boiled red beans, 50 g of sugar, 8 g of whole milk powder, 0.5 g of salt, 1.0 g of emulsifier (monoglyceride preparation), and 5.0 g (dry weight) of the cellulose composite powder composition were added to ion-exchanged water to adjust the total amount to 1,000 g. The resulting mixture was then charged into a food mixer to roughly crush the boiled red beans, and then using a piston type homogenizer (manufactured by APV, product name: "Manton Gaulin Homogenizer"), homogenization treatment was performed at 10 MPa to obtain a red bean milk (rough), and performed at 40 MPa 10 times to obtain a red bean milk (smooth). These were filled in a glass heat-resistant bottle having a volume of 200 mL, heat-sterilized (121°C, 30 minutes), and cooled with tap water for 1 hour. The cooled bottle was gently shaken up and down 10 times and then stored in an atmosphere of 5°C. for 1 month.

The suspension stability of each red bean milk after storage was visually observed to evaluate the separation state, the sedimentation state and oil-off. Also, the red bean milk after storage was drunk and the texture was sensory evaluated. The evaluation was performed for the separation state, sedimentation state, the number of re-dispersion and the texture based on the same criteria as for the carrot juice. The evaluation of oil-off was performed based on the following criteria. The evaluation results are shown in Tables 3 to 6.

(Oil ring)

The amount of the solidified oil confirmed along the edge of the bottle at the upper portion of the beverage in the heat-resistant bottle was evaluated.

◎ (excellent): no solidified oil
○ (good): solidified oil was slightly formed on a part of the edge of the bottle.
Δ (acceptable): solidified oil was formed in an incomplete ring shape on the edge of the bottle.
✕ (unacceptable): solidified oil was formed in a complete ring shape on the edge of the bottle.

[Table 3]

| Cellulose composite powder composition | | | A | | B | | C | |
|---|---|---|---|---|---|---|---|---|
| Homogenization process | | | 10 MPa | 40 MPa×10 | 10 MPa | 40 MPa×10 | 10 MPa | 40 MPa×10 |
| Carrot juice test results | Additive amount | | 0.15 | 0.15 | 0.20 | 0.20 | 0.20 | 0.20 |
| | Separation | | ◎ | ◎ | ◎ | ◎ | ◎ | ◎ |
| | Sedimentation | | ◎ | ◎ | ◎ | ◎ | ◎ | ◎ |
| | Redistribution count | | 0 | 0 | 0 | 0 | 0 | 0 |
| | Texture | | rough | smooth | rough | smooth | rough | smooth |
| Red bean milk test results | Additive amount | | 0.18 | 0.18 | 0.24 | 0.24 | 0.24 | 0.24 |
| | Separation | | ◎ | ◎ | ◎ | ◎ | ◎ | ◎ |
| | Sedimentation | | ◎ | ◎ | ◎ | ◎ | ◎ | ◎ |
| | Redistribution count | | 0 | 0 | 0 | 0 | 0 | 0 |
| | Oil ring | | ○ | ◎ | ◎ | ◎ | ◎ | ○ |
| | Texture | | rough | smooth | rough | smooth | rough | smooth |

[Table 4]

| Cellulose composite powder composition | | | D | | E | | F | |
|---|---|---|---|---|---|---|---|---|
| Homogenization process | | | 10 MPa | 40 MPa×10 | 10 MPa | 40 MPa×10 | 10 MPa | 40 MPa×10 |
| Carrot juice test results | Additive amount | | 0.25 | 0.25 | 0.275 | 0.275 | 0.3 | 0.3 |
| | Separation | | ◎ | ○ | ◎ | ○ | ◎ | △ |
| | Sedimentation | | ◎ | ○ | ◎ | △ | ◎ | △ |
| | Re-distribution number | | 0 | 1 | 0 | Five | 0 | 6 |
| | Texture | | rough | smooth | rough | smooth | rough | smooth |
| Red bean milk test results | Additive amount | | 0.3 | 0.3 | 0.33 | 0.33 | 0.36 | 0.36 |
| | Separation | | ◎ | ○ | ◎ | ○ | ◎ | △ |
| | Sedimentation | | ◎ | ○ | ○ | △ | ◎ | △ |
| | Re-distribution number | | 0 | 2 | 0 | 5 | 0 | 7 |
| | Oil ring | | ○ | △ | ◎ | ○ | ○ | △ |
| | Texture | | rough | smooth | rough | smooth | rough | smooth |

[Table 5]

| Cellulose composite powder composition | | G | | H | |
|---|---|---|---|---|---|
| Carrot juice Test results | Homogenization process | 10 MPa | 40 MPa×10 | 10 MPa | 40 MPa×10 |
| | Additive amount | 0.15 | 0.15 | 0.18 | 0.18 |
| | Separation | ◎ | ◎ | ◎ | ◎ |
| | Sedimentation | ◎ | ◎ | ◎ | ◎ |
| | Redistribution number | 0 | 0 | 0 | 0 |
| | Texture | rough | smooth | rough | smooth |
| Red bean milk Test results | Additive amount | 0.18 | 0.18 | 0.216 | 0.216 |
| | Separation | ◎ | ◎ | ◎ | ◎ |
| | Sedimentation | ◎ | ◎ | ◎ | ◎ |
| | Redistribution number | 0 | 0 | 0 | 0 |
| | Oil ring | ○ | ◎ | ◎ | ◎ |
| | Texture | rough | smooth | rough | smooth |

[Table 6]

| Cellulose composite powder composition | I | | J | | K | |
|---|---|---|---|---|---|---|
| Homogenization process | 10 MPa | 40 MPa×10 | 10 MPa | 40 MPa×10 | 10 MPa | 40 MPa×10 |
| **Carrot juice test results** | | | | | | |
| Additive amount | 0.5 | 0.5 | 0.2 | 0.2 | 0.15 | 0.15 |
| Separation | ◎ | × | ◎ | × | ◎ | △ |
| Sedimentation | △ | × | ◎ | × | ◎ | × |
| Redistribution number | 3 | 8 | 0 | Ten | 0 | 9 |
| Texture | rough | smooth | rough | smooth | rough | smooth |
| **Red bean milk test results** | | | | | | |
| Additive amount | 0.6 | 0.6 | 0.24 | 0.24 | 0.18 | 0.18 |
| Separation | ◎ | × | ◎ | × | ◎ | × |
| Sedimentation | △ | × | ◎ | × | ◎ | × |
| Redistribution number | 5 | 15 | 0 | 9 | 0 | 10 |
| Oil ring | ○ | × | ◎ | × | △ | × |
| Texture | rough | smooth | rough | smooth | rough | smooth |

When using any of the cellulose composite powder compositions, although the beverages homogenized at a low shear force (10 MPa) had a rough texture, the beverages homogenized at a high shear force (40 MPa for 10 times) had a smooth texture. On the contrary, it was observed that the suspension stability tended to be better in the beverage homogenized with a low shear force than the beverage homogenized with a high shear force, regardless of the type of the cellulose composite powder composition.

In the beverages containing the cellulose composite powder compositions I to K having the [G'2]/[G'1] of 0.45 to 0.49, although the beverages homogenized with a low shear force showed a relatively good suspension stability, the

29

beverages homogenized with a high shear force showed at least one of the separation, sedimentation and oil ring being evaluated as "×" (unacceptable), which means the suspension stability was poor. On the contrary, in the beverages containing the cellulose composite powder compositions A to H having the [G'2]/[G'1] of 0.5 or more, even in the beverages homogenized with a high shear force, all of the separation, sedimentation and oil ring were evaluated as "Δ" (acceptable) or higher, which means the suspension stability was excellent. It was observed that the larger the [G'2]/[G'1], the more excellent the suspension stability in the beverages homogenized with a high shear force. Above all, in the beverages containing the cellulose composite powder compositions A to C, G, and H having [G'2]/[G'1] of 0.90 or more, by homogenizing with a high shear force, beverages having excellent texture and good suspension stability were obtained. In particular, in the beverages containing the cellulose composite powder compositions A, B, G, and H having [G'2]/[G'1] of 1.0 or more, by homogenizing with a high shear force, beverages having both excellent texture and stability were obtained.

**Claims**

1. A cellulose composite comprising a cellulose and a polysaccharide, wherein
   a Bragg spacing by small-angle X-ray scattering (SAXS) analysis of a sediment obtained by subjecting a 0.1 % by mass aqueous dispersion of the cellulose composite to a centrifugation treatment is 8.6 nm or more.

2. The cellulose composite according to claim 1, wherein a ratio ([G'2]/[G'1]) between

   a storage elastic modulus (G'1) of an aqueous dispersion obtained by dispersing a mixture obtained by containing 1% by mass of the cellulose composite in ion-exchanged water using a high-shear homogenizer (manufactured by Nippon Seiki Co., Ltd., product name: "Excel Auto Homogenizer ED-7") at a rotation speed of 1,000 rpm for 5 minutes and
   a storage elastic modulus (G'2) of an aqueous dispersion obtained by dispersing the mixture using the high-shear homogenizer at a rotation speed of 16,000 rpm for 5 minutes
   is 0.5 or more.

3. The cellulose composite according to claim 1 or 2, wherein the storage elastic modulus (G'2) of the aqueous dispersion obtained by dispersing a mixture obtained by containing 1% by mass of the cellulose composite in ion-exchanged water using a high-shear homogenizer (manufactured by Nippon Seiki Co., Ltd., product name: "Excel Auto Homogenizer ED-7") at a rotation speed of 16,000 rpm for 5 minutes is 0.3 Pa or more.

4. The cellulose composite according to any one of claims 1 to 3, wherein

   the cellulose composite has a water content of 20% by mass or less, and
   the cellulose composite is in a powder form that passes through a sieve having an opening size of 1 mm.

5. A method for producing a cellulose composite including a cellulose and a polysaccharide, comprising
   compositing a hydrolyzate of a cellulose having a Bragg spacing of 7.5 nm or more with a polysaccharide.

6. A food and drink comprising the cellulose composite defined in any one of claims 1 to 4.

7. A pharmaceutical product comprising the cellulose composite defined in any one of claims 1 to 4.

8. An industrial product comprising the cellulose composite defined in any one of claims 1 to 4.

## FIG. 1

Legend:
- – – – – Example 1  LBKP  After Modifying Treatment
- ··········· Example 3  LBKP  After Modifying Treatment
- ——— LBKP  Before Modifying Treatment

## FIG. 2

Legend:
- – – – – Example 1  Sedimentation Part
- ——— Comparative Example 1  Sedimentation Part

## INTERNATIONAL SEARCH REPORT

| | International application No. |
|---|---|
| | PCT/JP2019/013956 |

**A. CLASSIFICATION OF SUBJECT MATTER**

Int.Cl.  A23L29/262(2016.01)i, A23L2/42(2006.01)i, A23L2/62(2006.01)i,
         A23L29/00(2016.01)i, A61K47/36(2006.01)i, A61K47/38(2006.01)i

According to International Patent Classification (IPC) or to both national classification and IPC

**B. FIELDS SEARCHED**

Minimum documentation searched (classification system followed by classification symbols)
Int.Cl.  A23L29/262, A23L2/42, A23L2/62, A23L29/00, A61K47/36, A61K47/38

Documentation searched other than minimum documentation to the extent that such documents are included in the fields searched

| | |
|---|---|
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)
CAplus/MEDLINE/EMBASE/BIOSIS (STN), FSTA (STN), JSTPlus/JMEDPlus/JST7580
(JDreamIII), PubMed, Japio-GPG/FX

**C. DOCUMENTS CONSIDERED TO BE RELEVANT**

| Category* | Citation of document, with indication, where appropriate, of the relevant passages | Relevant to claim No. |
|---|---|---|
| X | WO 2016/166798 A1 (ASAHI KASEI CORPORATION) 20 October 2016 claims 1, 5, paragraphs [0016], [0017], [0196]–[0206] & US 2018/0134878 A1, claims 1, 5, paragraphs [0019], [0020], [0279]–[0332] & EP 3284758 A1 | 1–8 |

☒ Further documents are listed in the continuation of Box C.　　☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 06 June 2019 (06.06.2019) | 18 June 2019 (18.06.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**INTERNATIONAL SEARCH REPORT**

International application No.

PCT/JP2019/013956

C (Continuation).    DOCUMENTS CONSIDERED TO BE RELEVANT

| Category* | Citation of document, with indication, where appropriate, of the relevant passages | Relevant to claim No. |
|---|---|---|
| X | JP 2015-074736 A (ASAHI KASEI CHEMICALS CORP.) 20 April 2015 claims 1, 4, paragraphs [0016], [0017], [0196]-[0203] (Family: none) | 1-8 |
| A | WO 2016/167269 A1 (ASAHI KASEI CORPORATION) 20 October 2016 entire text & US 2018/0110235 A1, entire text & EP 3284780 A1 | 1-8 |
| A | JP 2016-084397 A (ASAHI KASEI CHEMICALS CORP.) 19 May 2016 entire text (Family: none) | 1-8 |
| A | WO 2013/022090 A1 (ASAHI KASEI CHEMICALS CORP.) 14 February 2013 entire text & US 2014/0171521 A1, entire text & EP 2743277 A1 | 1-8 |
| A | WO 2011/125742 A1 (ASAHI KASEI CHEMICALS CORP.) 13 October 2011 entire text & US 2013/0022730 A1, entire text & EP 2554587 A1 | 1-8 |

Form PCT/ISA/210 (continuation of second sheet) (January 2015)

EP 3 777 565 A1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018071778 A
- WO 2013022090 A
- JP 2015502136 A